# EUROPÄISCHE PATENTSCHRIFT

(11) **EP 3 497 148 B1**
(45) Veröffentlichungstag und Bekanntmachung des Hinweises auf die Patenterteilung: **03.06.2020**
(21) Anmeldenummer: 17749478.8
(22) Anmeldetag: 09.08.2017
(51) Int. Cl.: C08G 65/26, C07C 309/29, C07C 309/35, C08G 63/82, C08G 67/04, C08G 69/20

(54) **VERFAHREN ZUR HERSTELLUNG VON POLYMEREN RINGÖFFNUNGSPRODUKTEN**
METHOD FOR THE PREPARATION OF POLYMER RING OPENING PRODUCTS
PROCEDE DE FABRICATION DE PRODUITS D'OUVERTURE DE CYCLE POLYMERES

(30) Priorität: 12.08.2016 EP 16184028; 28.02.2017 EP 17158356
(43) Veröffentlichungstag der Anmeldung: 19.06.2019
(73) Patentinhaber: Covestro Deutschland AG, 51373 Leverkusen (DE)
(72) Erfinder: GÜRTLER, Christoph, 50735 Köln (DE); MÜLLER, Thomas, Ernst, 52074 Aachen (DE); HOFMANN, Jörg, 47800 Krefeld (DE); LANGANKE, Jens, 53894 Mechernich (DE); LEVEN, Matthias, 51069 Köln (DE); LEITNER, Walter, 52074 Aachen (DE)
(74) Vertreter: Levpat
(86) Internationale Anmeldenummer: PCT/EP2017/070140
(87) Internationale Veröffentlichungsnummer: WO 2018/029232

(56) Entgegenhaltungen:
- WO-A1-2013/076177
- WO-A1-2015/155094
- US-A- 4 120 903
- US-A1- 2012 259 090
- US-B2- 9 080 013
- Esim Chemicals: "Monosodium Fumarate (MSF)", , 1. September 2015 (2015-09-01), XP055406378, Gefunden im Internet: URL:http://www.esim-chemicals.com/binaries /content/assets/products/dfc000048r1-msf-p roduct-data-sheet-esim.pdf [gefunden am 2017-09-13]
- Sigma-Aldrich: "Sodium dihydrogencitrate", , 1. Mai 2014 (2014-05-01), XP055406410, Gefunden im Internet: URL:http://www.sigmaaldrich.com/ [gefunden am 2017-09-13]
- Sigma Aldrich: "4-Amino-5-hydroxynaphthalene-2,7-disulfon ic acid monosodium salt hydrate", , 11. März 2013 (2013-03-11), XP055406430, Gefunden im Internet: URL:http://www.sigmaaldrich.com/ [gefunden am 2017-09-13]
- L. S. SKOROKHOD ET AL: "Synthesis and structure of Co(II), Ni(II), and Cu(II) complexes with Schiff bases, condensation products of 2-amino-4,8-naphthalenedisulfonic acid and aromatic carbinols", RUSSIAN JOURNAL OF INORGANIC CHEMISTRY., Bd. 52, Nr. 7, 1. Juli 2007 (2007-07-01), Seiten 1006-1012, XP055406502, GB ISSN: 0036-0236, DOI: 10.1134/S0036023607070066

## Beschreibung

Die vorliegende Erfindung betrifft ein Verfahren zur Anlagerung einer Verbindung (A) an eine H-funktionelle Starterverbindung (BH) in Gegenwart eines Katalysators, wobei die mindestens eine Verbindung (A) ausgewählt wird aus mindestens einer Gruppe bestehend aus Alkylenoxid (A-1), Lacton (A-2), Lactid (A-3), cyclisches Acetal (A-4), Lactam (A-5), cyclisches Anhydrid (A-6) und sauerstoffhaltige Heterocyclenverbindung (A-7) verschieden von (A-1), (A-2), (A-3), (A-4), und (A-6) wobei der Katalysator eine organische, n-protonige Bronsted-Säure (C) umfasst, wobei n ≥ 2 und Element der natürlichen Zahl und der Protolysegrad D 0<D<n ist mit n als maximale Zahl an übertragbaren Protonen und D als berechneter Protonenanteil der organischen, n-protonigen Bronsted-Säure (C). Weiterhin betrifft die Erfindung eine n-protonige Bronsted-Säure (C) mit einem Protolysegrad D 0<D<n, wobei n die maximale Zahl an übertragbaren Protonen mit n = 2, 3 oder 4 und D der berechnete Protonenanteil der organischen, n-protonigen Bronsted-Säure (C) ist.

Ein weiterer Gegenstand der vorliegenden Erfindung sind durch erfindungsgemäßes Verfahren erhältliche polymere Ringöffnungsprodukte, beispielsweise Polyole sowie daraus hergestellte Polyurethanpolymere.

Ein dritter Gegenstand der vorliegenden Erfindung sind n n-protonige Bronsted-Säuren (C) mit einem Protolysegrad D 0<D<n, wobei n die maximale Zahl an übertragbaren Protonen mit n = 2, 3 oder 4 und D der berechnete Protonenanteil der organischen, n-protonigen Bronsted-Säure (C) ist, wobei der Protolysegrad D für zweiprotonige Säuren mit n = 2 0,2 bis 1,9, für dreiprotonige Säuren mit n = 3 0,3 bis 2,8 und für vierprotonige Säuren mit n = 4 0,4 bis 3,7 beträgt.

Zum gegenwärtigen Zeitpunkt werden weltweit über 11 Millionen Tonnen Polyurethan pro Jahr produziert. Unter dem Aspekt einer nachhaltigeren Produktionsweise ist der Einsatz von zumindest teilweise aus nachwachsenden Rohstoffquellen stammenden Polyolen als Polyurethan-Rohstoffe erstrebenswert. Als C₁-Bausteine kommen insbesondere CO₂ und Formaldehyd in Frage (J. Langanke et al., Green Chem., 2014, 16, 1865-1870 und J. Polym. Sci. A: Polym. Chem., 2015, 53, 2071-2074). Diese sind nicht an die Verfügbarkeit von Erdöl gebunden und zudem preiswert.

Formaldehyd lässt sich zu Polyoxymethylen (POM) polymerisieren. Werden polymere Polyole als Starter verwendet, erhält man hierdurch Polyoxymethylen-Copolymere. Als Quellen für Formaldehyd dienen in der Regel gasförmiges Formaldehyd (monomer), Paraformaldehyd (polymer) und Trioxan (trimer). Die Reaktionsbedingungen der Polymerisation haben auch einen großen Einfluss auf die Struktur der so erhaltenen Block-Copolymere: die anionische Polymerisation von gasförmigem Formaldehyd in Gegenwart eines beispielsweise difunktionellen Starter-Polyols führt zu einer Triblock-Struktur, in der das Starter-Polyol zwei POM-Ketten trägt, welche den Abschluss des Block-Copolymers bilden. Es ist unter dem Aspekt der chemischen Stabilität vorteilhaft, wenn die terminale OH-Gruppe einer Polyoxymethylen-Einheit im Polymer derivatisiert wird. Dann wird der schrittweise Abbau der POM-Kette verhindert.

Die kationische Polymerisation von Alkylenoxiden wie beispielsweise Propylenoxid, Ethylenoxid an Hydroxylgruppen in Gegenwart von Lewis Säuren oder Bronsted Supersäuren wie beispielsweise HBH₄, HSbF₄, HPF₄, CF₃SO₃H und HClO₄ wird in "Chemistry of Polyols for Polyurethanes" von M. Ionescu wird in dem Abschnitt "Polyetherpolyols by Cationic Polymerisation Process" auf S. 245-246 beschrieben. Hierbei ist die Polymerisationsrate von Alkylenoxiden an H-funktionelle Starterverbindungen und hierbei speziell von Propylenoxiden auch bei niedrigen Temperaturen deutlich höher als bei der anionischen Polymerisation jedoch resultieren ungewünschte Nebenreaktionen wie die Bildung cyclischer Nebenprodukte wie beispielesweise Dioxane und Kronenether. Daher ist die säurenkatalysierte kationische Polymerisation aufgrund der Bildung des hohen Anteils von 15-25 % an cyclischen Oligomeren ungeeignet zur industriellen Herstellung von Polyetherpolyolen.

Die Verwendung von Borsäuresäureestern in Verbindung mit Haliden als Katalysator in der Ethoxylierung von basensenitiven Alkokolen wird in der Veröffentlichung von K. G. Moloy, Adv. Synth. Catal., 2010, 352, 821-826 beschrieben. Allerdings werden nur niedermolekulare Alkohole als Starterverbindungen ethoxyliert, wobei ein hoher Katalysatoranteil und lange Reaktionszeiten notwendig sind. Zudem gelten Borsäure und deren Ester als reproduktionstoxisch und erbgutverändernd.

In US 2012/0259090 A1 wird ein katalytisches Verfahren zur Copolymerisation von Ethylenoxid und Tetrahydrofuran beschrieben, wobei einer hoher Anteil an Nebenprodukte wie oligomeren cyclischen Ether gebildet wird. Der verwendete Katalysator besteht aus einer polymeren perfluorierten Sulfonsäure, die durch Copolymerisation von Tetrafluorethylen und CF2=CF-O-CF2CF(CF₃)-O-CF₂CF₂SO₂F und anschließende Hydrolyse hergestellt wird. Zur Konditionierung des polymeren Katalysators erfolgt eine fast 45 stündige Behandlung mit Wasser und Ethylenoxid und anschließende Trocknung. Weiterhin resultiert trotz einer verhältnismäßig hohen Katalysatorbeladung nur ein Teilumsatz des Ethylenoxids und Tetrahydrofurans in das entsprechende Copolymer. Es entstehen zudem Nebenprodukte.

Ein Verfahren zur Säure-katalysierten Polymerisation von Tetrahydrofuran wird in US 4,120,903 A beschrieben, wobei als Katalysator ein kommerzielles Polymer mit dem Handelsnamen Nafion®. Die Nafion®. Katalysatoren werden durch Coplymerisation von Tetrafluorethylen oder Hexafluorpropylen mit perfluorierten Sulfonsäureethern erhalten mit Äquivalentmassen von 943 bis 1500. Dies entspricht berechneten Molmassen der resultierenden Copolymere von 420682 g/mol bis 2910435 g/mol. Es resultiert jedoch trotz einer hohen Katalysatorbeladung und einer langen Reaktionszeit von 65 h jedoch nur ein Teilumsatz von 55,6 %. Nach Abtrennung des unreagierten Tetrahydrofurans erfolgt in einem zweiten Reaktionsschritt die Stabilisierung Polytetrahydrofurans durch Zugabe von 1,4-Butandiol.

Des Weiteren ist Nafion®. als stark saurer, polymerer Katalysator dafür bekannt, Umlagerungen von Alkylenoxiden zu katalysieren, wobei sich Epoxide unter Reaktionsbedingungen in entsprechende Aldyhyde und Ketone isomerisieren, was in Industrial & Engineering Chemistry Research, 2005, 44(23), 8468-8498 beschrieben wird.

In WO2015155094 (A1) wird ein Verfahren zur Herstellung von Polyoxymethylen-Blockcopolymeren beschrieben, umfassend den Schritt der Aktivierung des DMC-Katalysators in Anwesenheit einer OH-terminierten polymeren Formaldehyd-Starterverbindung mit einer definierten Menge Alkylenoxid, sowie einer optional anschließenden Polymerisation mit Alkylenoxiden und optional weiteren Co-Comonomeren. Hierbei wird die OH-terminierten polymeren Formaldehyd-Starterverbindung während der Aktivierungsphase bei möglichst milden Temperaturen mit Alkylenoxiden umgesetzt, um eine Depolymerisation der thermisch labilen bzw. metastabilen H-funktionellen Starterverbindung zu vermeiden. Die so gewonnenen Polyoxymethylen-Blockcopolymeren sind thermisch und chemisch stabil.

WO 2004/096746 A1 und US 2006/0205915 A1 offenbaren die Reaktion von Formaldehyd-Oligomeren mit Alkylenoxiden und/oder Isocyanaten. Bei dieser Methode werden über den beschriebenen Einsatz von Formaldehyd-Oligomeren HO-(CH₂O)ₙ-H Polyoxymethylen-Blockcopolymere mit einer relativ engen Molmassenverteilung von n = 2-19 erhalten, wobei für die Bereitstellung der Formaldehyd-Oligomere ausgehend von wässriger Formalinlösung ein zusätzlicher thermischer Abtrenn-Verfahrensschritt erforderlich ist. Die erhaltenen Formaldehyd-Oligomer-Lösungen sind hierbei nicht lagerstabil, so dass diese anschließend sofort weiterverarbeitet werden müssen.

Im Stand der Technik ist keine zufriedenstellende Methode zur Kettenverlängerung von chemisch bzw. thermisch labilen H-funktionellen Starterverbindungen, wie beispielsweise Polycarbonatpolyolen oder Polyacaetalverbindungen (z.B. Poly- bzw. para-Formaldehyd) mit Alkylenoxidverbindungen beschrieben, da für bekannte Katalysatorsysteme oftmals die Homopolymerisation als Nebenreaktion katalysiert wird. Des Weiteren resultiert für kationisch katalysierte Anlagerungen von Alkylenoxiden an H-funktionelle Starterverbindungen in Gegenwart von Bronsted-Säuren einer hoher Anteil an ungewünschten oliogmeren Nebenprodukten.

Aufgabe der vorliegenden Anmeldung war es verbesserte, nichtpolymere Katalysatorsysteme mit ausreichender Reaktivität bereitzustellen, welche die im Stand der Technik beschriebenen Nachteile reduzieren, um somit eine selektivere Umsetzung von vorzugsweise thermisch labilen H-funktionellen Starterverbindungen mit entsprechend reaktiven Verbindungen, speziell Alkylenoxiden, bei möglichst milden Reaktionsbedingungen zu kettenverlängerten Additionsprodukten, speziell zu kettenverlängerten Polyolen, zu realisieren und den Anteil an unerwünschten cyclischen Neben- und Abbauprodukten und/oder der Bildung von Isomerisierungsprodukten wie beispielsweise von Aldehyden oder Ketonen und deren ggf. auftretenden Folgeprodukten zu reduzieren. Es ist weiterhin wünschenswert, dass die verwendeten Katalysatoren schwermetallfrei sind und deren Vorstufen kommerziell verfügbar sind bzw. die Katalysatoren somit schnell und leicht synthetisierbar sowie konditionierbar sind.

Erfindungsgemäß gelöst wird die Aufgabe durch ein Verfahren zur Anlagerung einer Verbindung (A) an eine H-funktionelle Starterverbindung (BH) in Gegenwart eines Katalysators, wobei die mindestens eine Verbindung (A) ausgewählt wird aus mindestens einer Gruppe bestehend aus Alkylenoxid (A-1), Lacton (A-2), Lactid (A-3), cyclisches Acetal (A-4), Lactam (A-5), cyclisches Anhydrid (A-6) und sauerstoffhaltige Heterocyclenverbindung (A-7) verschieden von (A-1), (A-2), (A-3), (A-4) und (A-6) dadurch gekennzeichnet, dass der Katalysator eine organische, n-protonige Bronsted-Säure (C) umfasst, wobei n ≥ 2 und Element der natürlichen Zahl und der Protolysegrad D 0<D<n ist mit n als maximale Zahl an übertragbaren Protonen und D als berechneter Protonenanteil der organischen, n-protonigen Bronsted-Säure (C), wobei die organische, n-protonige Bronsted-Säure (C) eine berechnete Molmasse von ≤ 1200 g/mol aufweist.

In einer Ausführungsform des erfindungsgemäßen Verfahrens erfolgt die Anlagerung der Verbindung (A) an die H-funktionelle Starterverbindung (BH) in Gegenwart des Katalysators bevorzugt zu einem Ringöffnungsprodukt, bevorzugt zu einem polymeren Ringöffnungsprodukt, wobei hierunter die optional katalytisch induzierte Ringöffnung der Verbindung (A) im Zuge der Anlagerung an die H-funktionelle Starterverbindung (BH) und/oder an bereits angelagerte Ringöffnungsprodukte der Verbindung (A) zu verstehen ist.

In einer Ausführungsform des erfindungsgemäßen Verfahrens erfolgt die Anlagerung der Verbindung (A) an die H-funktionelle Starterverbindung (BH) in Gegenwart eines Katalysators, wobei die mindestens eine Verbindung (A) ausgewählt wird aus mindestens einer Gruppe bestehend aus Alkylenoxid (A-1), Lacton (A-2), Lactid (A-3), cyclisches Acetal (A-4), Lactam (A-5) und cyclisches Anhydrid (A-6), wobei der Katalysator eine organische, n-protonige Bronsted-Säure (C) umfasst, wobei n ≥ 2 und Element der natürlichen Zahl und der Protolysegrad D 0<D<n ist mit n als maximale Zahl an übertragbaren Protonen und D als berechneter Protonenanteil der organischen, n-protonigen Bronsted-Säure (C).

In einer bevorzugten Ausführungsform des erfindungsgemäßen Verfahrens erfolgt die Anlagerung der Verbindung (A) an die H-funktionelle Starterverbindung (BH) in Gegenwart des Katalysators, wobei die mindestens eine Verbindung (A) ausgewählt wird aus mindestens einer Gruppe bestehend aus Alkylenoxid (A-1), Lacton (A-2), cyclisches Acetal (A-4) und cyclisches Anhydrid (A-6), dadurch gekennzeichnet, dass der Katalysator eine organische, n-protonige Bronsted-Säure (C) umfasst, wobei n ≥ 2 und Element der natürlichen Zahl und der Protolysegrad D 0<D<n ist mit n als maximale Zahl an übertragbaren Protonen und D als berechneter Protonenanteil der organischen, n-protonigen Bronsted-Säure (C).

In einer alternativen Ausführungsform des erfindungsgemäßen Verfahrens erfolgt die Anlagerung der Verbindung (A) an die H-funktionelle Starterverbindung (BH) in Gegenwart eines Katalysators, wobei die mindestens eine Verbindung (A) ausgewählt wird aus mindestens einer Gruppe bestehend aus Alkylenoxid (A-1) und sauerstoffhaltige Heterocyclenverbindung (A-7) verschieden von (A-1), (A-2), (A-3), (A-4) und (A-6), wobei der Katalysator eine organische, n-protonige Bronsted-Säure (C) umfasst, wobei n ≥ 2 und Element der natürlichen Zahl und der Protolysegrad D 0<D<n ist mit n als maximale Zahl an übertragbaren Protonen und D als berechneter Protonenanteil der organischen, n-protonigen Bronsted-Säure (C).

### Verbindung (A)

Ist die Verbindung (A) ein Alkylenoxid, (A-1) kann dieses beispielsweise ein Epoxid mit 2-45 Kohlenstoffatomen sein. In einer bevorzugten Ausführungsform des Verfahrens wird das Alkylenoxid (A-1) ausgewählt aus mindestens einer Verbindung der Gruppe bestehend aus Ethylenoxid, Propylenoxid, 1-Butenoxid, 2,3-Butenoxid, 2-Methyl-1,2-propenoxid (Isobutenoxid), 1-Pentenoxid, 2,3-Pentenoxid, 2-Methyl-1,2-butenoxid, 3-Methyl-1,2-butenoxid, Epoxide von C6-C22 α-Olefinen, wie 1-Hexenoxid, 2,3-Hexenoxid, 3,4-Hexenoxid, 2-Methyl-1,2-pentenoxid, 4-Methyl-1,2-pentenoxid, 2-Ethyl-1,2-butenoxid, 1-Heptenoxid, 1-Octenoxid, 1-Nonenoxid, 1-Decenoxid, 1-Undecenoxid, 1-Dodecenoxid, 4-Methyl-1,2-pentenoxid, Cyclopentenoxid, Cyclohexenoxid, Cycloheptenoxid, Cyclooctenoxid, Styroloxid, Methylstyroloxid, Pinenoxid, Allylglycedylether, Vinylcyclohexenoxid, Cyclooctadienmonoepoxid, Cyclododecatrienmonoepoxid, Butadienmonoepoxid, Isoprenmonoepoxid, Limonenoxid, 1,4-Divinylbenzolmonoepoxid, 1,3-Divinylbenzolmonoepoxid, Glycidylacrylat und Glycidylmethacrylat ein- oder mehrfach epoxidierte Fette als Mono-, Di- und Triglyceride, epoxidierte Fettsäuren, C1-C24-Ester epoxidierter Fettsäuren, Epichlorhydrin, Glycidol, und Derivate des Glycidols wie beispielsweise Glycidylether von C1-C22 Alkanolen, Glycidylester von C1-C22 Alkancarbonsäuren. Beispiele für Derivate des Glycidols sind Phenylglycidylether, Kresylglycidylether, Methylglycidylether, Ethylglycidylether und 2-Ethylhexylglycidylether.

In einer besonders bevorzugten Ausführungsform des Verfahrens wird das Alkylenoxid (A-1) ausgewählt aus mindestens einer Verbindung der Gruppe bestehend aus Ethylenoxid, Propylenoxid Styroloxid und Allylglycidylether eingesetzt werden.

In einer bevorzugten Ausführungsform des Verfahrens wird das Lacton (A-2) ausgewählt aus mindestens einer Verbindung der Gruppe bestehend aus 4-gliedrigen Ringlactone wie Propiolacton, β-Butyrolacton, β-Isovalerolacton, β-Caprolacton, β-Isocaprolacton, β-Methyl-β-valerolacton, 5-gliedrigen Ringlactone, wie γ-Butyrolacton, γ-Valerolacton, 5-Methylfuran-2(3H)-on, 5-Methylidenedihydrofuran-2(3H)-on, 5-Hydroxyfuran-2(5H)-on, 2-Benzofuran-1(3H)-on und 6-Methy-2-benzofuran-1(3H)-on, 6-gliedrigen Ringlactone, wie δ-Valerolacton, 1,4-Dioxan-2-on, Dihydrocumarin, 1H-Isochromen-1-on, 8H-pyrano[3,4-b]pyridine-8-on, 1,4-Dihydro-3H-isochromen-3-on, 7,8-Dihydro-5H-pyrano[4,3-b]pyridine-5-on, 4-Methyl-3,4-dihydro-1H-pyrano[3,4-b]pyridine-1-on, 6-Hydroxy-3,4-dihydro-1H-isochromen-1-on, 7-Hydroxy-3,4-dihydro-2H-chromen-2-on, 3-Ethyl-1H-isochromen-1-on, 3-(Hydroxymethyl)-1H-isochromen-1-on, 9-Hydroxy-1H,3H-benzo[de]isochromen-1-on, 6,7-Dimethoxy-1,4-dihydro-3H-isochromen-3-on und 3-Phenyl-3,4-dihydro-1H-isochromen-1-on, 7-gliedrige Ringlactonen, wie ε-Caprolacton, 1,5-Dioxepan-2-on, 5-Methyloxepan-2-on, Oxepane-2,7-dion, thiepan-2-on, 5-Chlorooxepan-2-on, (4*S*)-4-(Propan-2-yl)oxepan-2-on, 7-Butyloxepan-2-on, 5-(4-Aminobuthyl)oxepan-2-on, 5-Phenyloxepan-2-on, 7-Hexyloxepan-2-on, (5*S*,7*S*)-5-Methyl-7-(propan-2-yl)oxepan-2-on, 4-Methyl-7-(propan-2-yl)oxepan-2-on, und höhergliedrige Ringlactone, wie (7*E*)-Oxacycloheptadec-7-en-2-on.

In einer besonders bevorzugten Ausführungsform des Verfahrens wird das Lacton (A-2) ausgewählt aus mindestens einer Verbindung der Gruppe bestehend ε-Caprolacton, Propiolacton, β-Butyrolacton, und γ-Butyrolacton.

In einer bevorzugten Ausführungsform des Verfahrens wird das Lactid (A-3) ausgewählt aus mindestens einer Verbindung der Gruppe bestehend aus Glycolid (1,4-Dioxan-2,5-dion), L-Lactid (L-3,6-Dimethyl-1,4-dioxan-2,5-dion), D-Lactid, DL-Lactid, Mesolactid und 3-Methyl-1,4-dioxan-2,5-dion, 3-Hexyl-6-methyl-1,4-dioxane-2,5-dione, 3,6-Di(but-3-en-1-yl)-1,4-dioxane-2,5-dion (jeweils einschließlich optisch aktiver Formen).

In einer bevorzugten Ausführungsform des Verfahrens wird das Lactid (A-3) ausgewählt aus mindestens einer Verbindung der Gruppe bestehend aus L-Lactid.

In einer bevorzugten Ausführungsform des Verfahrens wird das cyclisches Acetal (A-4) ausgewählt aus mindestens einer Verbindung der Gruppe bestehend aus 1,3,5-Trioxan, 1,3-Dioxan und 1,3-Dioxolan.

In einer bevorzugten Ausführungsform des Verfahrens wird das Lactam (A-5) ausgewählt aus mindestens einer Verbindung der Gruppe bestehend aus β-, γ-, δ- oder ε-Lactame.

Ist die Verbindung (A) ein cyclisches Anhydrid (A-6), kann dieses beispielsweise 2 bis 36, bevorzugt 2 bis 12 C-Atome enthalten. In einer bevorzugten Ausführungsform des Verfahrens wird das cyclische Anhydrid (A-6) ausgewählt aus mindestens einer Verbindung der Gruppe bestehend aus, 4-Cyclohexen-1,2-disäureanhydrid, 4-Methyl-4-cyclohexen-1,2-disäureanhydrid, 5,6-Norbornen-2,3-disäureanhydrid, Allyl-5,6-norbornen-2,3-disäureanhydrid, Dodecenylbernsteinsäureanhydrid, Tetradecenylbernsteinsäureanhydrid, Hexadecenyl-bernsteinsäureanhydrid, Octadecenylbernsteinsäureanhydrid, Succinsäureanhydrid, Maleinsäureanhydrid, Phthalsäureanhydrid, 1,2-Cyclohexandicarbonsäureanhydrid, Diphensäureanhydrid, Tetrahydrophthalsäureanhydrid, Methyltetra¬hydrophthalsäure¬anhydrid, Norbornendisäureanhydrid und ihre Chlorierungsprodukte, Bernsteinsäure¬anhydrid, Glutar¬isäure¬anhydrid, Diglykolsäureanhydrid, 1,8-Naphthalsäureanhydrid, Bernstein¬säureanhydrid, Dodecenylbernsteinsäureanhydrid, Tetra-decenyl¬bernsteinsäure¬anhydrid, Hexadecenylbernsteinsäure¬anhydrid, Octa¬decenyl-bernstein¬säureanhydrid, 3- und 4-Nitrophthalsäureanhydrid, Tetrachlorphthalsäure¬anhydrid, Tetrabromphthalsäureanhydrid, Itaconsäureanhydrid, Dimethylmaleinsäure¬anhydrid, Allylnorbornendisäureanhydrid, 3-Methylfuran-2,5-dion, 3-Methyldihydrofuran-2,5-dion, Dihydro-2H-pyran-2,6(3H)-dion, 1,4-Dioxane-2,6-dion, 2H-Pyran-2,4,6(3H,5H)-trion, 3-Ethyldihydrofuran-2,5-dion, 3-Methoxydihydrofuran-2,5-dion, 3-(Prop-2-en-1-yl)dihydrofuran-2,5-dion, N-(2,5-Dioxotetrahydrofuran-3-yl)formamid und 3[(2E)-But-2-en-1-yl]dihydrofuran-2,5-dion.

In einer besonders bevorzugten Ausführungsform des Verfahrens wird das cyclische Polycarbonsäureanhydrid (A-6) ausgewählt aus mindestens einer Verbindung der Gruppe bestehend aus Succinsäure¬anhydrid, Maleinsäureanhydrid, Itaconsäureanhydrid und Phthalsäureanhydrid.

Ist die Verbindung (A) eine sauerstoffhaltige Heterocyclenverbindung (A-7) verschieden von (A-1), (A-2), (A-3), (A-4) und (A-6), kann diese beispielsweise 3 bis 36, bevorzugt 3 bis 12 C-Atome im Ring enthalten.

In einer bevorzugten Ausführungsform des Verfahrens wird die sauerstoffhaltige Heterocyclenverbindung (A-7) verschieden von (A-1), (A-2), (A-3), (A-4) und (A-6) ausgewählt aus mindestens einer Verbindung der Gruppe bestehend aus unsubstituierten oder substituierten Oxetane, unsubstituierten oder substituierten Oxolane, unsubstituierten oder substituierten Oxane und unsubstituierten oder substituierten Oxepane.

In einer besonders bevorzugten Ausführungsform des Verfahrens wird die sauerstoffhaltige Heterocyclenverbindung (A-7) verschieden von (A-1), (A-2), (A-3), (A-4) und (A-6) ausgewählt aus mindestens einer Verbindung der Gruppe bestehend aus Trimethylenoxid, Furan, Tetrahydrofuran, Tetrahydropyran, Oxacycloheptatrien und Hexamethylenoxid.

In einer weiteren bevorzugten Ausführungsform des erfindungsgemäßen Verfahrens wird die mindestens eine Verbindung (A) ausgewählt aus der Gruppe bestehend aus Ethylenoxid, Propylenoxid, Styroloxid , Allylglycidylether, ε-Caprolacton, Propiolacton, β-Butyrolacton, γ-Butyrolacton, ε-Caprolactam, 1,3-Dioxolan, 1,4-Dioxan, Tetrahydrofuran und 1,3,5-Trioxan. In einer besonders bevorzugten Ausführungsform wird die die mindestens eine Verbindung (A) ausgewählt aus der Gruppe bestehend aus Ethylenoxid, Propylenoxid und β-Butyrolacton.

In einer weiteren bevorzugten Ausführungsform des erfindungsgemäßen Verfahrens wird die mindestens eine Verbindung (A) ausgewählt aus der Gruppe bestehend aus Ethylenoxid, Propylenoxid, Styroloxid , Allylglycidylether, ε-Caprolacton, Propiolacton, β-Butyrolacton, γ-Butyrolacton, ε-Caprolactam, 1,3-Dioxolan und 1,3,5-Trioxan. In einer besonders bevorzugten Ausführungsform wird die die mindestens eine Verbindung (A) ausgewählt aus der Gruppe bestehend aus Ethylenoxid, Propylenoxid und -Butyrolacton.

In einer weiteren alternativ bevorzugten Ausführungsform des erfindungsgemäßen Verfahrens wird die mindestens eine Verbindung (A) ausgewählt aus der Gruppe bestehend aus Ethylenoxid, Propylenoxid und Tetrahydrofuran.

### Verbindung (BH)

Als geeignete H-funktionelle Starterverbindungen (BH), auch als Starter bezeichnet, können Verbindungen mit für die Alkoxylierung aktiven H-Atomen eingesetzt werden. Für die Alkoxylierung aktive Gruppen mit aktiven H-Atomen sind beispielsweise -OH, -NH₂ (primäre Amine), -NH- (sekundäre Amine), -SH und -CO₂H, bevorzugt sind -OH und -NH₂, besonders bevorzugt ist -OH. Als H-funktionelle Starterverbindung können beispielsweise eine oder mehrere Verbindungen ausgewählt werden aus der Gruppe umfassend ein- oder mehrwertige Alkohole, mehrwertige Amine, mehrwertige Thiole, Aminoalkohole, Thioalkohole, Hydroxyester, Polyetherpolyole, Polyesterpolyole, Polyesteretherpolyole, Polyethercarbonatpolyole, Polycarbonatpolyole, Polycarbonate, Polyacetalen, polymere Formaldehydverbindungen, Polyethylenimine, Polyetheramine (z.B. sogenannte Jeffamine® von Huntsman, wie z.B. D-230, D-400, D-2000, T-403, T-3000, T-5000 oder entsprechende Produkte der BASF, wie z.B. Polyetheramin D230, D400, D200, T403, T5000), Polytetrahydrofurane (z.B. PolyTHF® der BASF, wie z.B. PolyTHF® 250, 650S, 1000, 1000S, 1400, 1800, 2000), Polytetrahydrofuranamine (BASF Produkt Polytetrahydrofuranamin 1700), Polyetherthiole, Polyacrylatpolyole, Ricinusöl, das Mono- oder Diglycerid von Ricinolsäure, Monoglyceride von Fettsäuren, chemisch modifizierte Mono-, Di- und/oder Triglyceride von Fettsäuren, und C1-C24 Alkyl-Fettsäureester, die im Mittel mindestens 2 OH-Gruppen pro Molekül enthalten. Beispielhaft handelt es sich bei den C1-C23 Alkyl-Fettsäureester, die im Mittel mindestens 2 OH-Gruppen pro Molekül enthalten, um Handelsprodukte wie Lupranol Balance® (Fa. BASF AG), Merginol®-Typen (Fa. Hobum Oleochemicals GmbH), Sovermol®-Typen (Fa. Cognis Deutschland GmbH & Co. KG) und Soyol®TM-Typen (Fa. USSC Co.).

In einer bevorzugten Ausführungsform des erfindungsgemäßen Verfahrens wird die Verbindung (BH) ausgewählt aus der Gruppe bestehend aus Polyetherpolyol, Polyethercarbonatpolyol und Polycarbonatpolyol. Hierbei erfolgt Herstellung des Polyethercarbonatpolyol und des Polycarbonatpolyols durch katalytische Anlagerung von Kohlenstoffdioxid und Alkylenoxiden an eine weitere H-funktionelle Starterverbindung. Bevorzugt erfolgt Herstellung des Polyethercarbonatpolyol und des Polycarbonatpolyols durch katalytische Anlagerung von Kohlenstoffdioxid und Ethylenoxid und/oder Propylenoxid an eine weitere H-funktionelle Starterverbindung. Als monofunktionelle Starterverbindungen können Alkohole, Amine, Thiole und Carbonsäuren eingesetzt werden. Als monofunktionelle Alkohole können Verwendung finden: Methanol, Ethanol, 1-Propanol, 2-Propanol, 1-Butanol, 2-Butanol, tert-Butanol, 3-Buten-1-ol, 3-Butin-l-ol, 2-Methyl-3-buten-2-ol, 2-Methyl-3-butin-2-ol, Propargylalkohol, 2-Methyl-2-propanol, 1-*tert*-Butoxy-2-propanol, 1-Pentanol, 2-Pentanol, 3-Pentanol, 1-Hexanol, 2-Hexanol, 3-Hexanol, 1-Heptanol, 2-Heptanol, 3-Heptanol, 1-Octanol, 2-Octanol, 3-Octanol, 4-Octanol, Phenol, 2-Hydroxybiphenyl, 3-Hydroxybiphenyl, 4-Hydroxybiphenyl, 2-Hydroxypyridin, 3-Hydroxypyridin, 4-Hydroxypyridin. Als monofunktionelle Amine kommen in Frage: Butylamin, *tert*-Butylamin, Pentylamin, Hexylamin, Anilin, Aziridin, Pyrrolidin, Piperidin, Morpholin. Als monofunktionelle Thiole können verwendet werden: Ethanthiol, 1-Propanthiol, 2-Propanthiol, 1-Butanthiol, 3-Methyl-l-butanthiol, 2-Buten-1-thiol, Thiophenol. Als monofunktionelle Carbonsäuren seien genannt: Ameisensäure, Essigsäure, Propionsäure, Buttersäure, Fettsäuren wie Stearinsäure, Palmitinsäure, Ölsäure, Linolsäure, Linolensäure, Benzoesäure, Acrylsäure.

Als H-funktionelle Starterverbindung geeignete mehrwertige Alkohole sind beispielweise zweiwertige Alkohole (wie beispielweise Ethylenglykol, Diethylenglykol, Propylenglykol, Dipropylenglykol, 1,3-Propandiol, 1,4-Butandiol, 1,4-Butendiol, 1,4-Butindiol, Neopentylglykol, 1,5-Pentantandiol, Methylpentandiole (wie beispielweise 3-Methyl-1,5-pentandiol), 1,6-Hexandiol; 1,8-Octandiol, 1,10-Decandiol, 1,12-Dodecandiol, Bis-(hydroxymethyl)-cyclohexane (wie beispielweise 1,4-Bis-(hydroxymethyl)cyclohexan), Triethylenglykol, Tetraethylenglykol, Polyethylenglykole, Dipropylenglykol, Tripropylenglykol, Polypropylenglykole, Dibutylenglykol und Polybutylenglykole); dreiwertige Alkohole (wie beispielweise Trimethylolpropan, Glycerin, Trishydroxyethylisocyanurat, Rizinusöl); vierwertige Alkohole (wie beispielsweise Pentaerythrit); Polyalkohole (wie beispielweise Sorbit, Hexit, Saccharose, Stärke, Stärkehydrolysate, Cellulose, Cellulosehydrolysate, hydroxyfunktionalisierte Fette und Öle, insbesondere Rizinusöl), sowie alle Modifizierungsprodukte dieser zuvor genannten Alkohole mit unterschiedlichen Mengen an ε-Caprolacton.

Die H-funktionellen Starterverbindungen können auch aus der Substanzklasse der Polyetherpolyole ausgewählt sein, insbesondere solchen mit einem Molekulargewicht Mₙ im Bereich von 100 bis 4000 g/mol. Bevorzugt sind Polyetherpolyole, die aus sich wiederholenden Ethylenoxid- und Propylenoxideinheiten aufgebaut sind, bevorzugt mit einem Anteil von 35 bis 100% Propylenoxideinheiten, besonders bevorzugt mit einem Anteil von 50 bis 100% Propylenoxideinheiten. Hierbei kann es sich um statistische Copolymere, Gradienten-Copolymere, alternierende oder Blockcopolymere aus Ethylenoxid und Propylenoxid handeln. Geeignete Polyetherpolyole, aufgebaut aus sich wiederholenden Propylenoxid- und/oder Ethylenoxideinheiten sind beispielsweise die Desmophen®-, Acclaim®-, Arcol®-, Baycoll®-, Bayfill®-, Bayflex®-Baygal®-, PET®- und Polyether-Polyole der Covestro AG (wie z. B. Desmophen® 3600Z, Desmophen® 1900U, Acclaim® Polyol 2200, Acclaim® Polyol 4000I, Arcol® Polyol 1004, Arcol® Polyol 1010, Arcol® Polyol 1030, Arcol® Polyol 1070, Baycoll® BD 1110, Bayfill® VPPU 0789, Baygal® K55, PET® 1004, Polyether® S180). Weitere geeignete homo-Polyethylenoxide sind beispielsweise die Pluriol® E-Marken der BASF SE, geeignete homo-Polypropylenoxide sind beispielsweise die Pluriol® P-Marken der BASF SE, geeignete gemischte Copolymere aus Ethylenoxid und Propylenoxid sind beispielsweise die Pluronic® PE oder Pluriol® RPE-Marken der BASF SE.

Die H-funktionellen Starterverbindungen können auch aus der Substanzklasse der Polyesterpolyole ausgewählt sein, insbesondere solchen mit einem Molekulargewicht Mₙ im Bereich von 200 bis 4500 g/mol. Als Polyesterpolyole können mindestens difunktionelle Polyester eingesetzt werden. Bevorzugt bestehen Polyesterpolyole aus alternierenden Säure- und Alkoholeinheiten. Als Säurekomponenten können z.B. Bernsteinsäure, Maleinsäure, Maleinsäureanhydrid, Adipinsäure, Phthalsäureanhydrid, Phthalsäure, Isophthalsäure, Terephthalsäure, Tetrahydrophtalsäure, Tetrahydrophthalsäureanhydrid, Hexahydrophthalsäureanhydrid oder Gemische aus den genannten Säuren und/oder Anhydride eingesetzt werden. Als Alkoholkomponenten werden z.B. Ethandiol, 1,2-Propandiol, 1,3-Propandiol, 1,4-Butandiol, 1,5-Pentandiol, Neopentylglykol, 1,6-Hexandiol, 1,4-Bis-(hydroxymethyl)-cyclohexan, Diethylenglykol, Dipropylenglykol, Trimethylolpropan, Glycerin, Pentaerythrit oder Gemische aus den genannten Alkoholen verwendet. Werden als Alkoholkomponente zweiwertige oder mehrwertige Polyetherpolyole eingesetzt, so erhält man Polyesteretherpolyole die ebenfalls als Starterverbindungen zur Herstellung der Polyethercarbonatpolyole dienen können. Bevorzugt werden Polyetherpolyole mit Mₙ = 150 bis 2000 g/mol zur Herstellung der Polyesteretherpolyole eingesetzt.

Des Weiteren können als H-funktionelle Starterverbindungen Polycarbonatdiole eingesetzt werden, insbesondere solche mit einem Molekulargewicht Mₙ im Bereich von 150 bis 4500 g/mol, vorzugsweise 500 bis 2500 g/mol, die beispielsweise durch Umsetzung von Phosgen, Dimethylcarbonat, Diethylcarbonat oder Diphenylcarbonat und difunktionellen Alkoholen oder Polyesterpolyolen oder Polyetherpolyolen hergestellt werden. Beispiele zu Polycarbonaten finden sich z.B. in der EP-A 1359177. Beispielsweise können als Polycarbonatdiole die Desmophen® C-

Typen der CovestroAG verwendet werden, wie z.B. Desmophen® C 1100 oder Desmophen® C 2200.

In einer weiteren Ausführungsform der Erfindung können Polyethercarbonatpolyole (z.B. cardyon® Polyole der Fa. Covestro), Polycarbonatpolyole (z.B. Converge® Polyole der Fa. Novomer / Saudi Aramco, NEOSPOL Polyole der Fa. Repsol etc.) und/oder Polyetherestercarbonatpolyole als H-funktionelle Starterverbindungen eingesetzt werden. Insbesondere können Polyethercarbonatpolyole, Polycarbonatpoylole und/oder Polyetherestercarbonatpolyole durch Umsetzung von Alkylenoxiden, bevorzugt Ethylenoxid, Propylenoxid oder deren Mischungen, optional weiteren Co-Monomeren mit CO₂ in Gegenwart einer weiteren H-funktionellen Starterverbindung und unter Verwendung von Katalysatoren erhalten werden. Diese Katalysatoren umfassen Doppelmetallcyanid-Katalysatoren (DMC-Katalysatoren) und/oder Metallkomplexkatalysatoren beispielsweise auf Basis der Metalle Zink und/oder Cobalt, wie beispielsweise Zink-Glutarat-Katalysatoren (beschrieben z.B. in M. H. Chisholm et al., Macromolecules 2002, 35, 6494), sogenannte Zink-Diiminat-Katalysatoren (beschrieben z.B. in S. D. Allen, J. Am. Chem. Soc. 2002, 124, 14284) und sogenannte Cobalt-Salen-Katalysatoren (beschrieben z.B. in US 7,304,172 B2, US 2012/0165549 A1) und/oder Mangan-Salen Komplexe. Eine Übersicht über die bekannten Katalysatoren für die Copolymerisation von Alkylenoxiden und CO₂ gibt zum Beispiel Chemical Communications 47(2011)141-163. Durch die Verwendung unterschiedlicher Katalysatorsysteme, Reaktionsbedingungen und/oder Reaktionssequenzen erfolgt hierbei die Bildung von statistischen, alternierenden, blockartigen oder gradientenartigen Polyethercarbonatpolyole, Polycarbonatpoylole und/oder Polyetherestercarbonatpolyole. Diese als H-funktionelle Starterverbindungen eingesetzten Polyethercarbonatpolyole, Polycarbonatpoylole und/oder Polyetherestercarbonatpolyole können hierzu in einem separaten Reaktionsschritt zuvor hergestellt werden.

Die H-funktionellen Starterverbindungen weisen im Allgemeinen eine OH-Funktionalität (d.h. Anzahl an für die Polymerisation aktiven H-Atomen pro Molekül) von 1 bis 8, bevorzugt von 2 bis 6 und besonders bevorzugt von 2 bis 4 auf. Die H-funktionellen Starterverbindungen werden entweder einzeln oder als Gemisch aus mindestens zwei H-funktionellen Starterverbindungen eingesetzt.

Bevorzugte H-funktionelle Starterverbindungen sind Alkohole mit einer Zusammensetzung nach der allgemeinen Formel (I),

HO-(CH₂)_{X}-OH (I)

wobei x eine Zahl von 1 bis 20, bevorzugt eine gerade Zahl von 2 bis 20 ist. Beispiele für Alkohole gemäß Formel (I) sind Ethylenglycol, 1,4-Butandiol, 1,6-Hexandiol, 1,8-Octandiol, 1,10 Decandiol und 1,12-Dodecandiol. Weitere bevorzugte H-funktionelle Starterverbindungen sind Neopentylglykol, Trimethylolpropan, Glycerin, Pentaerythrit, Umsetzungsprodukte der Alkohole gemäß Formel (VII) mit ε-Caprolacton, z.B. Umsetzungsprodukte von Trimethylolpropan mit ε-Caprolacton, Umsetzungsprodukte von Glycerin mit ε-Caprolacton, sowie Umsetzungsprodukte von Pentaerythrit mit ε-Caprolacton. Weiterhin bevorzugt werden als H-funktionelle Starterverbindungen Wasser, Diethylenglykol, Dipropylenglykol, Rizinusöl, Sorbit und Polyetherpolyole, aufgebaut aus sich wiederholenden Polyalkylenoxideinheiten, eingesetzt.

Besonders bevorzugt handelt es sich bei den H-funktionellen Starterverbindungen um eine oder mehrere Verbindungen ausgewählt aus der Gruppe bestehend aus Ethylenglykol, Propylenglykol, 1,3-Propandiol, 1,3-Butandiol, 1,4-Butandiol, 1,5-Pentandiol, 2-Methylpropan-1,3-diol, Neopentylglykol, 1,6-Hexandiol, Diethylenglykol, Dipropylenglykol, Glycerin, Trimethylolpropan, di- und trifunktionelle Polyetherpolyole, wobei das Polyetherpolyol aus einer di- oder tri-H-funktionellen Starterverbindung und Propylenoxid bzw. einer di- oder tri-H-funktionellen Starterverbindung, Propylenoxid und Ethylenoxid aufgebaut ist. Die Polyetherpolyole haben bevorzugt eine OH-Funktionalität von 2 bis 4 und ein Molekulargewicht Mₙ im Bereich von 62 bis 8000 g/mol, bevorzugt ein Molekulargewicht und mehr bevorzugt von ≥ 92 g/mol bis ≤ 2000 g/mol.

In einer weiteren Ausführungsform der Erfindung können Polyacetale, bevorzugt polymere Formaldehydverbindungen, als H-funktionelle Starterverbindungen für das erfindungsgemäße Verfahren eingesetzt werden, wobei sich grundsätzlich solche oligomeren und polymeren Formen des Formaldehyds eignen, welche wenigstens eine terminale Hydroxylgruppe zur Reaktion mit der Verbindung (A) aufweisen. Unter dem Begriff "terminale Hydroxylgruppe" wird erfindungsgemäß insbesondere eine terminale Halbacetal-Funktionalität verstanden, welche sich als Strukturmerkmal über die Polymerisation des Formaldehyds ergibt. Beispielsweise können die Starterverbindungen Oligomere und Polymere des Formaldehyds der allgemeinen Formel (I) sein, wobei n für eine ganze Zahl ≥ 2 steht und wobei polymeres Formaldehyd typischerweise n > 8 Wiederholungseinheiten aufweist.

Für das erfindungsgemäße Verfahren geeignete polymere Formaldehyd-Starterverbindungen weisen im Allgemeinen Molmassen von 62 bis 30000 g/mol, bevorzugt von 62 bis 12000 g/mol, besonders bevorzugt von 242 bis 6000 g/mol und ganz besonders bevorzugt von 242 bis 3000 g/mol auf und umfassen von 2 bis 1000, bevorzugt von 2 bis 400, besonders bevorzugt von 8 bis 200 und ganz besonders bevorzugt von 8 bis 100 Oxymethylen-Wiederholungseinheiten. Die im erfindungsgemäßen Verfahren eingesetzten Starterverbindungen haben typischerweise eine Funktionalität (F) von 1 bis 3, in bestimmten Fällen können diese aber auch höherfunktionell sein, also eine Funktionalität > 3 besitzen. Bevorzugt werden im erfindungsgemäßen Verfahren offenkettige polymere Formaldehyd-Starterverbindungen mit terminalen Hydroxylgruppen eingesetzt, welche eine Funktionalität von 1 bis 10, bevorzugt von 1 bis 5, besonders bevorzugt von 2 bis 3 haben. Ganz besonders bevorzugt werden im erfindungsgemäßen Verfahren lineare polymere Formaldehyd-Starterverbindungen eingesetzt, welche eine Funktionalität von 2 aufweisen (z.B. GRANUFORM® der Fa. Ineos). Die Funktionalität F entspricht der Anzahl an OH-Endgruppen pro Molekül.

Die Herstellung der polymeren Formaldehyd-Starterverbindungen, welche für das erfindungsgemäße Verfahren eingesetzt werden, kann nach bekannten Verfahren erfolgen (vgl. z.B. M. Haubs et. al., 2012, Polyoxymethylenes, Ullmann's Encyclopedia of Industrial Chemistry; G. Reus et. al., 2012, Formaldehyde, *ibid*). Die Formaldehyd-Starterverbindungen können im erfindungsgemäßen Verfahren grundsätzlich auch in Form eines Copolymers eingesetzt werden, wobei als Comonomere neben Formaldehyd beispielsweise 1,4-Dioxan, oder 1,3-Dioxolan einpolymerisiert sind. Weitere geeignete Formaldehyd-Copolymere für das erfindungsgemäße Verfahren sind Copolymere des Formaldehyds sowie des Trioxans mit cyclischen und/oder linearen Formalen, wie beispielsweise Butandiolformal, oder Epoxiden. Es ist ebenfalls denkbar, dass als Comonomere höhere homologe Aldehyde, wie beispielweise Acetaldehyd, Propionaldehyd, etc., in das Formaldehyd-Polymer eingebaut sind. Ebenfalls ist es denkbar, dass erfindungsgemäße Formaldehyd-Starterverbindungen wiederum ausgehend von H funktionellen Starterverbindungen hergestellt werden, insbesondere lassen sich hierbei durch den Einsatz von mehrwertigen Starterverbindungen polymere Formaldehyd-Starterverbindungen mit einer Hydroxy-EndgruppenFunktionalität F > 2 erhalten (vgl. z.B. WO 1981001712 A1, Bull. Chem. Soc. J., 1994, 67, 2560-2566, US 3436375, JP 03263454, JP 2928823).

Für das erfindungsgemäße Verfahren können auch Gemische unterschiedlicher polymerer Formaldehyd-Starterverbindungen oder Gemische mit anderen H-funktionellen Starterverbindungen eingesetzt werden. Als geeignete H-funktionelle Starterverbindung ("Starter") können Verbindungen mit für die Alkoxylierung aktiven H-Atomen eingesetzt werden, die eine Molmasse von 18 bis 4500 g/mol, bevorzugt von 62 bis 2500 g/mol und besonders bevorzugt von 62 bis 1000 g/mol aufweisen. Für die Alkoxylierung aktive Gruppen mit aktiven H-Atomen sind beispielsweise -OH, -NH₂ (primäre Amine), -NH- (sekundäre Amine), -SH und -CO₂H, bevorzugt sind -OH und -NH₂, besonders bevorzugt ist -OH. Als H-funktionelle Starterverbindung wird beispielsweise eine oder mehrere Verbindungen ausgewählt aus der Gruppe bestehend aus ein- oder mehrwertigen Alkoholen, mehrwertigen Aminen, mehrwertigen Thiolen, Aminoalkohole, Thioalkohole, Hydroxyester, Polyetherpolyole, Polyesterpolyole, Polyesteretherpolyole, Polyethercarbonatpolyole, Polycarbonatpolyole, Polycarbonate, Polyethylenimine, Polyetheramine, Polytetrahydrofurane (z. B. PolyTHF® der BASF), Polytetrahydrofuranamine, Polyetherthiole, Polyacrylatpolyole, Ricinusöl, das Mono- oder Diglycerid von Ricinolsäure, Monoglyceride von Fettsäuren, chemisch modifizierte Mono-, Di- und/oder Triglyceride von Fettsäuren, und C1-C24 Alkyl-Fettsäureester, die im Mittel mindestens 2 OH-Gruppen pro Molekül enthalten, eingesetzt.

Bekanntlich polymerisiert Formaldehyd bereits durch die Gegenwart geringer Wasserspuren. In wässriger Lösung bildet sich daher in Abhängigkeit von Konzentration und Temperatur der Lösung ein Gemisch von Oligomeren und Polymeren unterschiedlicher Kettenlängen, welche mit molekularem Formaldehyd und Formaldehyd-Hydrat im Gleichgewicht stehen. Sogenanntes Paraformaldehyd fällt hierbei als ein weißer, schlecht-löslicher Feststoff, aus der Lösung aus und stellt in der Regel ein Gemisch linearer Formaldehyd-Polymere mit n = 8 bis 100 Oxymethylen-Wiederholungseinheiten dar.

Ein Vorteil des erfindungsgemäßen Verfahrens liegt insbesondere darin, dass polymeres Formaldehyd bzw. sogenanntes Paraformaldehyd, welches kommerziell und kostengünstig erhältlich ist und darüber hinaus einen geringen CO2-Fussabdruck (engl. carbon footprint) aufweist, direkt als Starterverbindung eingesetzt werden kann, ohne dass hierbei zusätzliche vorbereitende Schritte nötig sind. In einer vorteilhaften Ausführungsform der Erfindung wird daher Paraformaldehyd als Starterverbindung eingesetzt. Insbesondere lassen sich über das Molekulargewicht und die Endgruppenfunktionalität der polymeren Formaldehyd-Starterverbindung Plolyoxymethylen-Blöcke mit definiertem Molgewicht und Funktionalität in das Produkt einbringen.

Vorteilhafterweise kann hierbei im erfindungsgemäßen Verfahren die Länge des Polyoxymethylen-Blocks einfach über das Molekulargewicht der eingesetzten Formaldehyd-Starterverbindung gesteuert werden. Vorzugsweise werden hierbei lineare Formaldehyd-Starterverbindungen der allgemeinen Formel (I), wobei n für eine ganze Zahl ≥ 2 steht, bevorzugt mit n= 2 bis 1000, besonders bevorzugt mit n = 2 bis 400 und ganz besonders bevorzugt mit n = 8 bis 100, mit zwei terminalen Hydroxylgruppen eingesetzt. Insbesondere können als Starterverbindung auch Gemische von polymeren Formaldehyd-Verbindungen der Formel (I) mit jeweils unterschiedlichen Werten für n eingesetzt werden. In einer vorteilhaften Ausführungsform enthalten die eingesetzten Gemische von polymeren Formaldehyd-Starterverbindungen der Formel (I) wenigstens 1 Gew-%, bevorzugt wenigstens 5 Gew.-% und besonders bevorzugt wenigstens 10 Gew.-% an polymeren Formaldehyd-Verbindungen mit n ≥ 20.

In einer Ausführungsform des erfindungsgemäßen Verfahrens beträgt das molare Verhältnis der Verbindung (A) zu der H-funktionellen Starterverbindung (BH) im resultierenden Additionsproduktes ≥1, bevorzugt 1 bis 200 besonders bevorzugt 1-150.

### n-protonige Bronsted-Säure (C)

Im Sinne der vorliegenden Erfindung bezeichnet der Begriff der organischen, n-protonigen Bronsted-Säure (C) eine organische, nicht-polymere Verbindung, die aus Kohlenstoff, Wasserstoff und Sauerstoff sowie optional eines weiteren Heteroatoms ungleich der vorgenannten Atome, bevorzugt enthaltend eines weiteren Heteroatoms, aufgebaut ist, wobei die organische, n-protonige Bronsted-Säure (C) eine berechnete Molmasse von ≤ 1200 g/mol aufweist. Hierbei sind unter Bronsted-Säuren entsprechend der gängigen, fachlichen Definition Stoffe zu verstehen, welche Protonen an einen zweiten Reaktionspartner, die sogenannte Bronsted-Base typischerweise im wässrigen Medium bei 25 °C übertragen können. Die maximale Zahl an übertragbaren Protonen n beträgt im erfindungsgemäßen Verfahren n ≥ 2, bevorzugt n = 2, 3 oder 4 besonders bevorzugt n =2 oder 3 ganz besonders bevorzugt n = 2, wobei n Element der natürlichen Zahlen ist. Der berechnete Protonenanteil der organischen, n-protonigen Bronsted-Säure (C) entspricht dem Protolysegrad D und beträgt im erfindungsgemäßen Verfahren 0<D<n. Für zweiprotonige Säuren mit n = 2 beträgt der Protolysegrad D bevorzugt 0,2 bis 1,9 besonders bevorzugt 0,5 bis 1,8 und ganz besonders bevorzugt 0,8 bis 1,8. Für dreiprotonige Säuren mit n = 3 beträgt der Protolysegrad D bevorzugt 0,3 bis 2,8 besonders bevorzugt 0,7 bis 2,5 und ganz besonders bevorzugt 1,1 bis 2,1. Für vierprotonige Säuren mit n = 4 beträgt der Protolysegrad D bevorzugt 0,4 bis 3,7 besonders bevorzugt 0,8 bis 3,5 und ganz besonders bevorzugt 1,1 bis 3,1.

In Ausführungsform betrifft die Erfindung ein Verfahren wobei die Verbindung (C) eine nicht-polymere Verbindung ist.

In Ausführungsform betrifft die Erfindung ein Verfahren, wobei die organische, n-protonige Bronsted-Säure (C) eine berechnete Molmasse von ≤ 1200 g/mol, bevorzugt von ≤ 1000 g/mol und besonders bevorzugt von ≤ 850 g/mol aufweist.

In Ausführungsform betrifft die Erfindung ein Verfahren, wobei die organische, n-protonige Bronsted-Säure (C) eine berechnete Molmasse von ≥ 90 g/mol, bevorzugt von ≥ 100 g/mol und besonders bevorzugt von ≥ 110 g/mol aufweist.

In Ausführungsform betrifft die Erfindung ein Verfahren, wobei die organische, n-protonige Bronsted-Säure (C) eine berechnete Molmasse von ≥ 90 g/mol bis ≤ 1200 g/mol, bevorzugt von ≥90 g/mol bis ≤ 1000 g/mol und besonders bevorzugt von ≥100 g/mol bis ≤ 850 g/mol aufweist.

In einer Ausführungsform des erfindungsgemäßen Verfahrens ist die organische, n-protonige Bronsted-Säure (C) eine Sulfonsäure, wobei unter einer Sulfonsäure gemäß der gängigen fachlichen Definition eine organische Schwefelverbindungen mit der allgemeinen Struktur R-SO₂-OH, wobei R ein organischer Rest ist. Hierbei entspricht R-SO₂-OH oder abgekürzt R-SO₃H der Sulfonsäure sowie R-SO₃⁽⁻⁾ der deprotonierten Sulfonatgruppe, welche durch Protonenabgabe an einen zweiten Reaktionspartner die sogenannte Bronsted-Base, gebildet wird. Der organische Rest R entspricht hierbei linearen, cyclischen oder linear verzweigten Alkylengruppe mit 1 bis 22 Kohlenstoffatomen optional enthaltend weitere Heteroatome und/oder Arylgruppen mit 5 bis 18 Kohlenstoffatomen optional enthaltend weitere Heteroatome.

In einer bevorzugten Ausführungsform werden substituierte oder unsubstituierte aromatische nichtpolymere Polysulfonsäuren mit einer maximalen Zahl an übertragbaren Protonen n von n = 2, 3 oder 4 verwendet. In einer besonders bevorzugten Ausführungsform werden substituierte oder unsubstituierte Naphthalin-polysulfonsäuren mit n = 2 oder 3 und/oder substituierte oder unsubstituierte Benzolpolysulfonsäuren mit n = 2 oder 3 verwendet. Ganz besonders bevorzugt werden 1,5-Naphthalindisulfonsäuren, 2,6-Naphthalindisulfonsäuren und/oder 1,3-Benzoldisulfonsäuren mit n = 2. Für zweiprotonige Sulfonsäuren mit n = 2 beträgt der Protolysegrad D bevorzugt 0,2 bis 1,9 besonders bevorzugt 0,5 bis 1,8 und ganz besonders bevorzugt 0,8 bis 1,8. Für dreiprotonige Sulfonsäuren mit n = 3 beträgt der Protolysegrad D bevorzugt 0,3 bis 2,8 besonders bevorzugt 0,7 bis 2,5 und ganz besonders bevorzugt 1,1 bis 2,1. Für vierprotonige Sulfonsäuren mit n = 4 beträgt der Protolysegrad D bevorzugt 0,4 bis 3,7 besonders bevorzugt 0,8 bis 3,5 und ganz besonders bevorzugt 1,1 bis 3,1.

In Ausführungsform betrifft die Erfindung ein Verfahren, wobei die Sulfonsäure eine berechnete Molmasse von ≥ 160 g/mol bis ≤ 1200 g/mol, bevorzugt von ≥ 200 g/mol bis ≤ 1000 g/mol und besonders bevorzugt von ≥ 230 g/mol bis ≤ 850 g/mol aufweist.

In einer Ausführungsform des erfindungsgemäßen Verfahrens wird die organische, n-protonige Bronsted-Säure (C) mit dem Protolysegrad 0<D<n durch Säure-Base-Reaktionen mit Protonenübergang durch
(a) Zugabe geeigneter Mengen von geeigneten Bronsted-Basen (E) zu den organischen, n-protonigen Bronsted-Säuren oder
(β) Zugabe geeigneter Mengen von geeigneten Bronsted-Säuren (E'H) zu den Salzen der organischen n-protonigen Bronsted-Säuren erhalten.

### Schritt α

In einer bevorzugten Ausführungsform von Schritt (α) wird die organische, n-protonige Bronsted-Säure (C) mit dem Protolysegrad 0<D<n durch Säure-Base-Reaktionen mit Protonenübergang durch Zugabe geeigneter Mengen einer geeigneten Bronsted-Base (E) zu der vollständig oder partiell protonierten organischen, n-protonigen Bronsted-Säure erhalten, wobei eine geeignete Bronsted-Base (E) eine größere Basenstärke K_{b}(E) und damit einen einen kleineren pK_{b}(E)-Wert als die vollständig oder partiell protonierte organische, n-protonigen Bronsted-Säure aufweist. Die geeignete Menge an der Bronsted-Base (E) leitet sich beispielsweise aus dem zu erzielenden Protolysegrad (D) der organischen, n-protonigen Bronsted-Säure (C) und/oder der Basenstärke K_{b}(E) der verwendeten Bronsted-Base (E) ab.

Beispielhaft kann eine solche organische, n-protonigen Bronsted-Säure (C) mit einem Protolysegrad (D) im Bereich 0<D<n durch Umsetzung vollständig oder partiell protonierten, organischen, n-protonigen Bronsted-Säuren, bevorzugt vollständig protonierten Sulfonsäuren durch Zugabe von Bronsted-Basen (E) mit einem pK_{b}(E)-Wert von ≤10, bevorzugt mit einem pK_{b}(E)-Wert von ≤8 und ganz besonders bevorzugt mit einem pK_{b}(E)-Wert von ≤5 erhalten werden. Besonders bevorzugt werden Disulfonsäuren mit Metallorthovanadaten, Metallwolframaten, Metallhydrogentriphosphaten, Metallhydrogenphospaten, Metallsulfiten, Metalltrithiocarbonaten, Metallcarbonaten, Metallhydrogencarbonaten, Alkalimetallhydroxiden, Erdalkalimetallhydroxiden, Titanhydroxid, Zinkhydroxid, Aluminiumhydroxid, Vanadium- und Vanadylhydroxide, Eisen(II)- und Eisen(III)hydroxide, Bismut(III)hydroxid, Galium(III)hydroxid, Kupfer(II)hydroxid, Mangan(II)hydroxid, Silberhydroxid, Ammoniumhydroxide, Phosphoniumhydroxide, Sulfoniumhydroxiden, Sulfoxoniumhydroxiden, Alkalimetallalkoholatebn, Erdalkalimetallalkoholaten, Titanalkoholaten, Zinkalkoholaten, Aluminiumalkoholat, Ammoniumalkoholaten, Phosphoniumalkoholaten, Sulfoniumalkoholate, Sulfoxoniumalkoholaten, Alkalimetallalkoxylaten, Erdalkalimetallalkoxylaten, Titanalkoxylaten, Zinkalkoxylaten, Aluminiumalkoxylat, Ammoniumalkoxylaten, Phosphoniumalkoxylaten, Sulfoniumalkoxylaten, Sulfoxoniumalkoxylaten, Metallbenzoaten, Metallacetaten, Metallphenolaten, Hydrazin, Methyl- und Ethylhydrazin, Metallaziden, Hydroxylamin, Ammoniak, substituierten aliphatischen und cycloaliphatischen Mono-/Di-/Triaminen mit primären, sekundären oder tertiären AminFunktionen, Lithiumdiisopropylamid, Lithiumorganyle, Amidinbasen, Amide, Alkalimetallhydride, Erdalkalimetallhydride, Titanhydride, Zinkhydride, Aluminiumhydrid, Ammoniumhydride, Phosphoniumhydride, Sulfoniumhydride und/oder Sulfoxoniumhydride als Bronsted-Base (E) zu der organische, n-protonigen Bronsted-Säure (C) mit einem Protolysegrad (D) im Bereich 0<D<n umgesetzt. Ganz besonders bevorzugt werden Benzol-1,3-dilsulfonsäure, Naphthalin-1,5-dilsulfonsäure und/oder Naphthalin-2,6-dilsulfonsäure mit Lithiumhydroxiden, Natriumhydroxiden, Kaliumhydroxiden, Rubidiumhydroxiden, Cäsiumhydroxiden, Magnesiumhydroxiden, Calciumhydroxiden, Strontiumhydroxiden, Bariumhydroxiden, Scandiumhydroxiden, Titanhydroxiden, Zinkhydroxiden, Aluminiumhydroxiden, aliphatischen primären Ammoniumhydroxiden, aliphatischen sekundären Ammoniumhydroxiden, aliphatischen tertiären Ammoniumhydroxiden, aliphatischen quaternären Ammoniumhydroxiden, Phosphoniumhydroxiden, aliphatischen primären Ammoniumalkoholaten, aliphatischen sekundären Ammoniumalkoholaten, aliphatischen tertiären Ammoniumalkoholaten, aliphatischen quaternären Ammoniumalkoholaten, Phosphoniumalkoholaten, Butyllithium, Kalium-*tert-*butanolat, 1,8-Diazabicyclo[5.4.0]undec-7-en (DBU), 1,5-Diazabicyclo(4.3.0)non-5-en (DBN) und/oder primäre, sekundäre und tertiäre aliphatischen und cycloaliphatischen Mono- und Diaminen, bevorzugt mit Natriumhydroxid, Kaliumhydroxid, Lithiumhydroxid, Magnesiumhydroxid Calciumhydroxid, aliphatischen quaternären Ammoniumalkoholaten, Phosphoniumalkoholaten, Ammoniak, Triethylamin, Trimethylamin, Diethylamin, Propylamin, Methylamin, Dimethylamin, Ethylamin, Ethylenediamin, 1,3-Diaminpropane, Putrescin, 1,5-Diaminopentan, Hexamethylendiamin, 1,2-Diaminopropane, Diaminocyclohexan, n-Propylamin, Di-n-Propylamin, Tri-n-Propylamin, Isopropylamin, Diisopropylamin, n-Butylamin, Di-n-butylamin, Tri-n-butylamin, Di-isobutylamin, 2-Aminobutan, 2-Ethylhexylamin, Di-2-Ethylhexylamin, Cyclohexylamin, Dicyclohexylamin, Dimethylaminopropylamin Triethylendiamin, 1,8-Diazabicyclo[5.4.0]undec-7-en (DBU), 1,5-Diazabicyclo(4.3.0)non-5-en (DBN) besonders bevorzugt mit Natriumhydroxid, Kaliumhydroxid, Lithiumhydroxid, Magnesiumhydroxid Tetra-(n-Butyl)ammoniummethanolat, Tetra-(n-Butyl)ammoniumethanolat und/oder Tetra-(n-Butyl)ammoniumisopropylat als Bronsted-Basen (E) zu den organische, n-protonigen Bronsted-Säure (C) mit einem Protolysegrad (D) im Bereich 0<D<n umgesetzt.

Infolge der Säure-Base-Reaktionen durch Zugabe der Bronsted-Base (E) zu der vollständig oder partiell protonierten organischen, n-protonigen Bronsted-Säure erfolgt neben der Bildung der organischen, n-protonigen Bronsted-Säure (C) mit 0<D<n die Bildung der korrespondierenden Bronsted-Säure (EH) durch Protonenaufnahme, wobei bevorzugt Alkohole oder Wasser und ganz bevorzugt Wasser, Methanol Isopropanol oder Ethanol als korrespondierende Bronsted-Säuren (EH) gebildet werden.

In einer besonders bevorzugten Ausführungsform von (α) erfolgt nach der Bildung der organischen, n-protonigen Bronsted-Säure (C) in Schritt (α) eine Separation oder keine Separation der korrespondierenden Bronsted-Säure (EH), welche durch Protonenaufnahme aus der Bronsted-Base (E) gebildet wird. Bevorzugt wird hierbei die korrespondierende Bronsted-Säure (EH) separiert durch Destillation.

### Schritt β

In einer bevorzugten Ausführungsform von Schritt (β) wird die organischen, n-protonigen Bronsted-Säure (C) mit dem Protolysegrad 0<D<n durch Säure-Base-Reaktionen mit Protonenübergang durch einer geeigneten Bronsted-Säure (E'H) zu den Salzen der partiell oder vollständig deprotonierten organischen n-protonigen Bronsted-Säure, wobei eine geeignete Bronsted-Säure (E'H) eine größere Säurestärke Kₛ(E'H) und damit einen kleineren pKₛ(E'H)-Wert als die vollständig oder partiell deprotonierten organischen, n-protonigen Bronsted-Säure aufweist. Die geeignete Menge an der Bronsted-Säure (E'H) leitet sich beispielsweise aus dem zu erzielenden Protolysegrad (D) der organischen, n-protonigen Bronsted-Säure (C) und/oder der Säurestärke Kₛ(E'H) der verwendeten Bronsted-Säure (E'H) ab.

Beispielhaft kann eine solche organische, n-protonigen Bronsted-Säure (C) mit einem Protolysegrad (D) im Bereich 0<D<n durch Umsetzung eines vollständig oder partiell deprotonierten Metallsalzes einer organischen, n-protonigen Bronsted-Säure, bevorzugt eines Metallsalzes einer Sulfonsäure durch Zugabe starker Bronsted-Säure (E'H) mit einem pK_{S}(E'H)-Wert von ≤1 erhalten werden erhalten. Besonders bevorzugt werden Dialkalimetallsalze und/oder Diammoniumsalzes einer Disulfonsäure mit starken organischen und/oder anorganischen Bronsted-Säuren (E'H) mit einem pKₛ(E'H)-Wert von ≤1 zu den organische, n-protonigen Bronsted-Säure (C) mit einem Protolysegrad (D) im Bereich 0<D<n umgesetzt. Ganz besonders bevorzugt werden die Dinatrium und/oder die Dikaliumsalze der Benzol-1,3-dilsulfonsäure, Naphthalin-1,5-dilsulfonsäure und/oder Naphthalin-2,6-dilsulfonsäure mit aliphatischen und aromatischen fluorierten Sulfonsäuren, Trifluoromethansulfonsäure, Perchlorsäure, Chlorwasserstoffsäure, Bromwasserstoffsäure, Jodwasserstoffsäure, Fluorsulfonsäure, Bis(trifluoromethan)sulfonimid, Hexafluorantimonsäure, Pentacyanocyclopentadien, Pikrinsäure, Schwefelsäure, Salpetersäure, Trifluoressigsäure, Methansulfonsäure, Paratoluolsulfonsäure, aromatische Sulfonsäuren, aliphatische Sulfonsäuren, bevorzugt mit Trifluoromethansulfonsäure, Perchlorsäure, Chlorwasserstoffsäure, Bromwasserstoffsäure, Jodwasserstoffsäure, Fluorsulfonsäure, Bis(trifluoromethan)sulfonimid, Hexafluorantimonsäure, Pentacyanocyclopentadien, Pikrinsäure, Schwefelsäure, Salpetersäure, Trifluoressigsäure, Methansulfonsäure, Paratoluolsulfonsäure, Methansulfonsäure, Paratoluolsulfonsäure und besonders bevorzugt mit Trifluoromethansulfonsäure, Perchlorsäure, Chlorwasserstoffsäure, Bromwasserstoffsäure, Jodwasserstoffsäure, Bis(trifluoromethan)sulfonimid, Pentacyanocyclopentadien, Schwefelsäure, Salpetersäure, Trifluoressigsäure zu den organische, n-protonigen Bronsted-Säure (C) mit einem Protolysegrad (D) im Bereich 0<D<n umgesetzt.

Infolge der Säure-Base-Reaktionen durch Zugabe der Bronsted-Säure (E'H) zu der vollständig oder partiell deprotonierten organischen, n-protonigen Bronsted-Säure erfolgt neben der Bildung der organischen, n-protonigen Bronsted-Säure (C) mit 0<D<n die Bildung der korrespondierenden Bronsted-Base (E') durch Protonenabgabe, wobei bevorzugt Metallsalze, Phosphoniumsalze, oder Ammoniumsalze und besonders bevorzugt Alkalimetallsalze oder Ammoniumsalze und ganz bevorzugt Natriumhyhydrogensulfat, Kaliumhydorgensulfat, Lithiumhydrogensulfat, Natriumtriflat, Kaliumtriflat, Lithiumtriflat und/oder Ammoniumtriflat als korrespondierende Bronsted-Säure (EH) gebildet werden.

In einer besonders bevorzugten Ausführungsform erfolgt nach der Bildung der organischen, n-protonigen Bronsted-Säure (C) in Schritt (β) eine Separation der korrespondierenden Bronsted-Base (E') oder in keine Separation der korrespondierenden Bronsted-Base (E'), welche durch Protonenabgabe aus der Bronsted-Base (E') gebildet wird. Bevorzugt wird hierbei die korrespondierende Bronsted-Base (E') nicht separiert.

In einer weiteren Ausführungsform des Verfahrens wird die organische, n-protonige Bronsted-Säure (C) in einer Menge von 0,0001 mol-% bis 10 mol-%, bezogen auf die Stoffmenge der Verbindung (BH) eingesetzt.

In einer weiteren Ausführungsform des erfindungsgemäßen Verfahrens wird die Anlagerung der Verbindung (A) an die H-funktionelle Starterverbindung (BH) in Gegenwart der organischen, n-protonigen Bronsted-Säure (C) unter möglichst milden Reaktionstemperaturen von 20 °C bis 180 °C durchgeführt, um beispielsweise Zersetzungsreaktionen oder unerwünschte Nerbenreaktionen von chemisch bzw. thermisch labilen H-funktionellen Starterverbindungen (BH), wie beispielsweise Polycarbonatpolyolen oder Polyacaetalverbindungen zu reduzieren. Die Umsetzung der Verbindung (BH) mit (A) in Gegenwart der organischen, n-protonigen Bronsted-Säure (C) kann isotherm, adiabat optional mit definiertem Temperaturprofil erfolgen.

In einer weiteren Ausführungsform des erfindungsgemäßen Verfahrens wird die Anlagerung der Verbindung (A) an die H-funktionelle Starterverbindung (BH) in Gegenwart der organischen, n-protonigen Bronsted-Säure (C) in Reaktionszeiten bis 24 h bevorzugt bis 20 h, besonders bevorzugt bis 12 h durchgeführt.

In einer weiteren Ausführungsform kann das Verfahren in Batch-Fahrweise, Semi-Batch Fahrweise, oder kontinuierlicher Fahrweise durchgeführt werden.

In einer weiteren Ausführungsform des erfindungsgemäßen Verfahrens wird die Anlagerung der Verbindung (A) an die H-funktionelle Starterverbindung (BH) in Gegenwart der organischen, n-protonigen Bronsted-Säure (C) lösungsmittelfrei oder in einem Lösungsmittel durchgeführt. Bevorzugte Lösungsmittel sind polare oder unpolare aprotische Lösungsmittel. Besonders bevorzugt sind Lösungsmittel ausgewählt aus der Gruppe bestehend aus cyclischen Propylencarbonat, Ethylacetat, *iso*-Propylacetat, *n*-Butylacetat, Tetrahydrofuran, 1,3-Dioxolan, 1,3,5-Trioxepane, 1,4-Dioxan, Dimethyldioxan, Methylphenylether und Toluol.

In einer weiteren Ausführungsform des erfindungsgemäßen Verfahrens kann die organische, n-protonige Bronsted-Säure (C) auch direkt in Anwesenheit des Starters (BH) in Anwesenheit oder Abwesenheit eines weiteren Lösungsmittels hergestellt werden. Bevorzugt werden hierbei chlorierte Lösungsmittel und besonders bevorzugt Dichlormethan und/oder Chloroform verwendet.

In Ausführungsform betrifft die Erfindung eine n-protonige Bronsted-Säure (C), wobei die n-protonige Bronsted-Säure (C) eine berechnete Molmasse von ≥ 90 g/mol bis ≤ 1200 g/mol, bevorzugt von ≥ 100 g/mol bis ≤ 1000 g/mol und besonders bevorzugt von ≥ 110 g/mol bis ≤ 850 g/mol aufweist.

Gegenstand der Erfindung ist auch ein Ringöffnungsproduktes, bevorzugt eines mehrwertigen Alkohols, mehrwertigen Amins, mehrwertigen Thiols, Aminoalkohols, Thioalkohols, Hydroxyesters, Polyetherpolyols, Polyesterpolyols, Polyesteretherpolyols, Polyethercarbonat-polyols, Polycarbonatpolyols, Polycarbonats, polymeren Formaldehydverbindung, Polyamids, erhältlich durch ein Verfahren wie auf den vorangegangenen Seiten beschrieben.

Ein weiterer Aspekt der Erfindung ist ein Polyurethanpolymer, welches durch Reaktion umfassend des erfindungsgemäßen Ringöffnungsproduktes, bevorzugt eines mehrwertigen Alkohols, mehrwertigen Amins, mehrwertigen Thiols, Aminoalkohols, Thioalkohols, Hydroxyesters, Polyetherpolyols, Polyesterpolyols, Polyesteretherpolyols, Polyethercarbonat-polyols, Polycarbonatpolyols, Polycarbonats, polymeren Formaldehydverbindung, Polyamids mit einer Isocyanatkomponente umfassend ein Polyisocyanat erhältlich ist.

Das Isocyanat kann ein aliphatisches oder aromatisches Di- oder Polyisocyanat sein. Beispiele sind 1,4-Butylendiisocyanat, 1,5-Pentandiisocyanat, 1,6-Hexamethylendiisocyanat (HDI) bzw. deren Dimere, Trimere, Pentamere, Heptamere oder Nonamere oder Gemische derselben, Isophorondiisocyanat (IPDI), 2,2,4- und/oder 2,4,4-Trimethylhexamethylendiisocyanat, die isomeren Bis(4,4'-isocyanatocyclohexyl)methane oder deren Mischungen beliebigen Isomerengehalts, 1,4-Cyclohexylendiisocyanat, 1,4-Phenylendiisocyanat, 2,4- und/oder 2,6-Toluylendiisocyanat (TDI), 1,5-Naphthylendiisocyanat, 2,2'-und/oder 2,4'- und/oder 4,4'-Diphenylmethandiisocyanat (MDI) und/oder höhere Homologe (polymeres MDI), 1,3- und/oder 1,4-Bis-(2-isocyanato-prop-2-yl)-benzol (TMXDI), 1,3-Bis-(isocyanatomethyl)benzol (XDI), sowie Alkyl-2,6-diisocyanatohexanoate (Lysindiisocyanate) mit C1 bis C6-Alkylgruppen. Bevorzugt ist hierbei ein Isocyanat aus der Diphenylmethandiisocyanatreihe.

Neben den vorstehend genannten Polyisocyanaten können anteilig auch modifizierte Diisocyanate mit Uretdion-, Isocyanurat-, Urethan-, Carbodiimid, Uretonimin, Allophanat-, Biuret-, Amid-, Iminooxadiazindion- und/oder Oxadiazintrionstruktur sowie nicht-modifiziertes Polyisocyanat mit mehr als 2 NCO-Gruppen pro Molekül wie zum Beispiel 4-Isocyanatomethyl-1,8-octandiisocyanat (Nonantriisocyanat) oder Triphenylmethan-4,4',4"-triisocyanat mit eingesetzt werden.

Es ist möglich, dass das Isocyanat ein Prepolymer ist, welches erhältlich ist durch Reaktion eines Isocyanats mit einer NCO-Funktionalität von ≥ 2 und Ringöffnungsprodukten, bevorzugt eines mehrwertigen Alkohols, mehrwertigen Amins, mehrwertigen Thiols, Aminoalkohols, Thioalkohols, Hydroxyesters, Polyetherpolyols, Polyesterpolyols, Polyesteretherpolyols, Polyethercarbonat-polyols, Polycarbonatpolyols, Polycarbonats, polymeren Formaldehydverbindung, Polyamids und mit einem Molekulargewicht von ≥ 62 g/mol bis ≤ 8000 g/mol und OH-Funktionalitäten von ≥ 1,5 bis ≤ 6.

Ein weiterer Gegenstand der Erfindung ist eine n-protonige Bronsted-Säure (C) mit einem Protolysegrad D 0<D<n, wobei n die maximale Zahl an übertragbaren Protonen mit n = 2, 3 oder 4 und D der berechnete Protonenanteil der organischen, n-protonigen Bronsted-Säure (C) ist, dadurch gekennzeichnet, dass Säuren mit n = 2 0,2 bis 1,9, für dreiprotonige Säuren mit n = 3 0,3 bis 2,8 und für vierprotonige Säuren mit n = 4 0,4 bis 3,7 beträgt, wobei die organische, n-protonige Bronsted-Säure (C) mit dem Protolysegrad 0<D<n durch Säure-Base-Reaktionen mit Protonenübergang durch
(α) Zugabe geeigneter Mengen von mindestens einer geeigneten Bronsted-Base (E) zu der mindestens einen organischen, n-protonigen Bronsted-Säure erhalten wird, wobei die Bronsted-Base (E) mindestens ein Kation (F) enthält, das ausgewählt ist aus der Gruppe bestehend aus Alkalimetall-, Erdalkalimetall-, Halbmetall-haltige-, Übergangsmetall-, Lanthanoidmetall-, aliphatische Ammonium - haltige und Phosphonium - haltige und Sulfonium - haltige - Kationen
   oder
(β) Zugabe geeigneter Mengen von mindestens einer geeigneten Bronsted-Säure (E'H) zu dem Salz der mindestens einen organischen n-protonigen Bronsted-Säure erhalten wird, wobei die Salze der organischen n-protonigen Bronsted-Säure mindestens ein Kation (F') enthält, das ausgewählt ist aus der Gruppe bestehend aus Alkalimetall-, Erdalkalimetall-, Halbmetall-haltige, Übergangsmetall-haltige, Lanthanoidmetall-haltige, aliphatische Ammonium - haltige und Phosphonium - haltige und Sulfonium - haltige - Kationen, wobei die n-protonige Bronsted-Säure (C) mindestens eine Sulfonsäure ist und wobei die mindestens eine Sulfonsäure ausgewählt wird aus der Gruppe bestehend aus 1,5-Naphthalindisulfonsäure, 2,6-Naphthalindisulfonsäure und/ 1,3-Benzoldisulfonsäure, bevorzugt 1,5-Naphthalindisulfonsäure, 2,6-Naphthalindisulfonsäure und ganz besonders bevorzugt 2,6-Naphthalindisulfonsäure.

In einer Ausführung der erfindungsgemäßen n-protonigen Bronsted-Säure (C) wird das Kation (F) ausgewählt wird aus der Gruppe bestehend aus Lithiumkation, Natriumkation, Kaliumkation, Rubidiumkation, Cäsiumkation, Magnesiumkation, Calciumkation, Strontiumkation, Bariumkation, Scandiumkation, Titankation, Zinkkation, Aluminiumkation, aliphatischen primären Ammonium-Ionen, aliphatischen sekundären Ammonium-Ionen, aliphatischen tertiären Ammonium-Ionen, aliphatischen quaternären Ammonium-Ionen, Phosphonium Ionen, Sulfonium-Ionen und Sulfoxonium Ionen, bevorzugt aus Lithiumkation, Natriumkation, Kaliumkation, Magnesiumkation, Calciumkation, quaternären Ammonium-Ionen, und Triphenylphosphonium Ionen und besonders bevorzugt aus Natriumkation, Kaliumkation, Magnesiumkation und n-Butyl-Ammoniumion.

In einer Ausführung der erfindungsgemäßen n-protonigen Bronsted-Säure (C) wird das Kation (F') ausgewählt wird aus der Gruppe bestehend aus Lithiumkation, Natriumkation, Kaliumkation, Rubidiumkation, Cäsiumkation, Magnesiumkation, Calciumkation, Strontiumkation, Bariumkation, Scandiumkation, Titankation, Zinkkation, Aluminiumkation, aliphatischen primären Ammonium-Ionen, aliphatischen sekundären Ammonium-Ionen, aliphatischen tertiären Ammonium-Ionen, aliphatischen quaternären Ammonium-Ionen, Phosphonium Ionen, Sulfonium-Ionen und Sulfoxonium Ionen, bevorzugt aus Lithiumkation, Natriumkation, Kaliumkation, Magnesiumkation, Calciumkation, quaternären Ammonium-Ionen, und Triphenylphosphonium Ionen und besonders bevorzugt aus Natriumkation, Kaliumkation, Magnesiumkation und n-Butyl-Ammoniumion.

In einer Ausführung der erfindungsgemäßen n-protonigen Bronsted-Säure (C) wird die mindestens eine Bronsted-Base (E) ausgewählt ist aus der Gruppe bestehend aus Lithiumhydroxid, Natriumhydroxid, Kaliumhydroxid, Rubidiumhydroxid, Cäsiumhydroxid, Magnesiumhydroxid, Calciumhydroxid, Strontiumhydroxid, Bariumhydroxid, Scandiumhydroxid, Titanhydroxid, Zinkhydroxid, Aluminiumhydroxid, aliphatischen primären Ammoniumhydroxiden, aliphatischen sekundären Ammoniumhydroxiden, aliphatischen tertiären Ammoniumhydroxiden, aliphatischen quaternären Ammoniumhydroxiden, Phosphoniumhydroxiden, aliphatischen primären Ammoniumalkoholaten, aliphatischen sekundären Ammoniumalkoholaten, aliphatischen tertiären Ammoniumalkoholaten, aliphatischen quaternären Ammoniumalkoholaten, Phosphoniumalkoholaten, Butyllithium, Kalium-tert-butanolat, 1,8-Diazabicyclo[5.4.0]undec-7-en (DBU), 1,5-Diazabicyclo(4.3.0)non-5-en (DBN), primären aliphatischen Aminen, sekundären aliphatischen Aminen, tertiären aliphatischen Aminen, primären cycloaliphatischen Aminen, sekundären cycloaliphatischen Aminen, tertiären cycloaliphatischen Aminen und Phosphoniumalkoholaten bevorzugt aus Natriumhydroxid, Kaliumhydroxid, Lithiumhydroxid, Magnesiumhydroxid Calciumhydroxid, aliphatischen quaternären Ammoniumalkoholaten, Phosphoniumalkoholaten, Ammoniak, Triethylamin, Trimethylamin, Diethylamin, Propylamin, Methylamin, Dimethylamin, Ethylamin, Ethylenediamin, 1,3-Diaminpropane, Putrescin, 1,5-Diaminopentan, Hexamethylendiamin, 1,2-Diaminopropane, Diaminocyclohexan, n-Propylamin, Di-n-Propylamin, Tri-n-Propylamin, Isopropylamin, Diisopropylamin, n-Butylamin, Di-n-butylamin, Tri-n-butylamin, Di-isobutylamin, 2-Aminobutan, 2-Ethylhexylamin, Di-2-Ethylhexylamin, Cyclohexylamin, Dicyclohexylamin, Dimethylaminopropylamin Triethylendiamin, 1,8-Diazabicyclo[5.4.0]undec-7-en (DBU) und 1,5-Diazabicyclo(4.3.0)non-5-en (DBN) besonders bevorzugt aus Natriumhydroxid, Kaliumhydroxid, Lithiumhydroxid, Magnesiumhydroxid, Tetra-(n-Butyl)ammoniummethanolat, Tetra-(n-Butyl)ammoniumethanolat und Tetra-(n-Butyl)ammoniumisopropylat.

In einer Ausführung der erfindungsgemäßen n-protonigen Bronsted-Säure (C) wird die mindestens eine Bronsted-Säure (E'H) ausgewählt ist aus der Gruppe bestehend aus aliphatischen fluorierten Sulfonsäuren, aromatischen fluorierten Sulfonsäuren, Trifluoromethansulfonsäure, Perchlorsäure, Chlorwasserstoffsäure, Bromwasserstoffsäure, Jodwasserstoffsäure, Fluorsulfonsäure, Bis(trifluoromethan)sulfonimid, Hexafluorantimonsäure, Pentacyanocyclopentadien, Pikrinsäure, Schwefelsäure, Salpetersäure, Trifluoressigsäure, Methansulfonsäure, Paratoluolsulfonsäure, aromatische Sulfonsäuren und aliphatische Sulfonsäuren, bevorzugt aus Trifluoromethansulfonsäure, Perchlorsäure, Chlorwasserstoffsäure, Bromwasserstoffsäure, Jodwasserstoffsäure, Fluorsulfonsäure, Bis(trifluoromethan)sulfonimid, Hexafluorantimonsäure, Pentacyanocyclopentadien, Pikrinsäure, Schwefelsäure, Salpetersäure, Trifluoressigsäure, Methansulfonsäure, Paratoluolsulfonsäure, Methansulfonsäure und Paratoluolsulfonsäure besonders bevorzugt aus Trifluoromethansulfonsäure, Perchlorsäure, Chlorwasserstoffsäure, Bromwasserstoffsäure, Jodwasserstoffsäure, Bis(trifluoromethan)sulfonimid, Pentacyanocyclopentadien, Schwefelsäure, Salpetersäure und Trifluoressigsäure.

In einer Ausführung der erfindungsgemäßen n-protonigen Bronsted-Säure (C) wird eine zweiprotonige Säuren mit n = 2 mit einem Protolysegrad D von 0,8 bis 1,8, bevorzugt 1,1 bis 1,7, verwendet.

In einer Ausführung ist die n-protonige Bronsted-Säure (C) mindestens eine Sulfonsäure.

In einer Ausführung der erfindungsgemäßen n-protonigen Bronsted-Säure (C) wird die Sulfonsäure ausgewählt aus der Gruppe bestehend aus mindestens einer substituierten oder unsubstituierten Naphthalin-polysulfonsäure mit n = 2 oder 3 und/oder mindestens einer substituierte oder unsubstituierten Benzolpolysulfonsäure mit n = 2 oder 3.

Die Erfindung betrifft ferner eine Sulfonsäure wie in einem der Ansprüche 10 bis 14 definiert, wobei der Protolysegrad D 0,8 bis 1,8, bevorzugt 1,1 bis 1,7, beträgt.

### Beispiele

Die vorliegende Erfindung wird anhand der nachfolgenden Figuren und Beispiele näher erläutert, ohne jedoch darauf beschränkt zu sein.

### Verbindungen (A)

Ethylenoxid (3.0, Reinheit ≥ 99,9 Gew.-%, Linde AG)
Propylenoxid (Reinheit 99,9 %, Chemogas GmbH)
Styroloxid (Reinheit 97 %, Sigma-Aldrich Chemie GmbH)
Allylglycidylether (Reinheit 99 %, Sigma-Aldrich Chemie GmbH)
1,3-Dioxolan (Reinheit 99,8 %, Sigma-Aldrich Chemie GmbH)
β-Butyrolacton (Reinheit 98 %, Sigma-Aldrich Chemie GmbH)
ε-Caprolacton (Reinheit 97 %, Sigma-Aldrich Chemie GmbH)
Maleinsäureanhydrid (Reinheit 99 %, Sigma-Aldrich Chemie GmbH)
Tetrahydrofuran (absolut, Reinheit 99,9 %, Sigma-Aldrich Chemie GmbH)

### Verbindungen (BH) mit mindestens einem Zerewitinoff-aktivem Wasserstoffatom

### PEG:

Polyethylenglykol mit einer Molekularmasse von 1000 g/mol wurde vom Hersteller *Fluka* bezogen. Vor der weiteren Verwendung wurde das kommerziell erhältliche Produkt im Exsikkator über Phosphorpentoxid getrocknet.

### PET:

Polypropylenglykol (ARCOL® POLYOL 1004) mit einer gemittelten Molekularmasse von 432 g/mol (Hydroxyzahl (OH-Zahl): 250-270 mg(KOH)/g) wurde vom Hersteller Covestro AG bezogen. Vor der weiteren Verwendung wurde das erhältliche Produkt im Hochvakuum getrocknet.

PC: CONVERGE® Polyol 212-10, M=1000 g/mol:
Polycarbonatdiol der Fa. Novomer Inc. CONVERGE® Polyol 212-10 erhältlich durch Umsetzung von CO2 und Propylenoxid mit einer durchschnittlichen Molekularmasse von 1000 g/mol, einem CO2-Anteil von ca. 40 Gew.-%, einer OH-Zahl von 112 mg(KOH)/g Eine Untersuchung des Ausgangsstoffs mittels Protonenresonanzspektroskopie erwies einen Gehalt von 3 Gew.-% cyclischem Propylencarbonat (cPC).
pFA: Paraformaldehyd (M=450 g/mol)
Paraformaldehyd (Markenname: Granuform 96) wurde vom Hersteller *Ineos* AG bezogen. Die zahlenmittlere Molekularmasse des Produkts wird mit 450 g/mol angegeben.

### Verwendete Katalysatoren bzw. deren Ausgangsstoffe

1,3-Benzoldisulfonsäure-dinatriumsalz: 1,3-Na2-BDS (Reinheit 94 %, Sigma-Aldrich Chemie GmbH)
1,5-Naphtalindisulfonsäure-dinatriumsalz: 1,5-Na2-NDS (Reinheit 95 %, Sigma-Aldrich Chemie GmbH)
2,6-Naphtalindisulfonsäure-dinatriumsalz: 2,6-Na2-NDS (Reinheit 97 %, Sigma-Aldrich Chemie GmbH)
1,5-Naphthalindisulfonsäure 1,5-NDS (Reinheit, 97 %, Sigma-Aldrich Chemie GmbH)
Schwefelsäure: H₂SO₄ (Reinheit 98 %, Sigma-Aldrich Chemie GmbH)
Trifluormethansulfonsäure: CF₃SO₃H (Reinheit 98 %, Sigma-Aldrich Chemie GmbH)
Natriumtriflat: NaOTf (Reinheit 97 %, Sigma-Aldrich Chemie GmbH)
Natriumhydrogensulfat: NaHSO₄ (Reinheit 90 %, Sigma-Aldrich Chemie GmbH)
Para-Toluolsulfonsäure: p-TSA (Reinheit 98 %, Sigma-Aldrich Chemie GmbH)
Fumarsäure C₄H₄O₄: (Reinheit 99 %, Sigma-Aldrich Chemie GmbH)
Tetrabutylammoniummetoxid Lösung: (n-Bu)₄N (OMe) (20% ige Methanollösung, Sigma-Aldrich Chemie GmbH)
1,8-Diazabicyclo[5.4.0]undec-7-en: DBU (Reinheit 98 %, Sigma-Aldrich Chemie GmbH)
Perfluorsulfonsäure Membran: Typ Nafion®. N117 in einer Dicke von 0,01778 cm (0.007 Inch) mit einer Äquivalentmenge an Sulfonylgruppen von 0.91-1.11 mmol/g (Herstellerangaben für Ionenaustauschkapazität IEC), Sigma-Aldrich Chemie GmbH

### Beschreibung der Methoden:

Gel-Permeations-Chromatographie (GPC): Die Messungen erfolgten auf einem Agilent 1200 Series (G1310A Iso Pump, G1329A ALS, G1316A TCC, G1362A RID, G1365D MWD), Detektion über RID; Elutionsmittel: Tetrahydrofuran (GPC grade), Flussrate 1.0 ml/min; Säulenkombination: PSS SDV Vorsäule 8×50 mm (5 µm), 2× PSS SDV linear S 8×300 ml (5 µm). Polystyrol bekannter Molmasse der Firma "PSS Polymer Standards Service" wurden zur Kalibrierung verwendet. Als Messaufnahme- und Auswertungssoftware wurde das Programmpaket "PSS WinGPC Unity" verwendet. Die Aufnahme der GPC Chromatogramme erfolgte in Anlehnung an DIN 55672-1. Das Peak-Molekulargewicht (kurz: MPeak oder M_{P}) entspricht laut GPC Chromatogram dem Molgewicht, entsprechend der Kalibrierung, bei maximalem Detektorsignal.

Der Polydispersitätsindex aus gewichtetem (M_{w}) und Zahlenmittlerem (Mₙ) Molekulargewicht der Gel-Permeations-Chromatographie ist definiert als M_{w}/Mₙ.

¹H-NMR-Spektroskopie (Protonenresonanzspektroskopie): Die Messungen erfolgten auf einem Bruker AV400 (400 MHz); die Kalibrierung der chemischen Verschiebungen erfolgte relativ zum Lösungsmittelsignal (CDCl₃, δ = 7,26 ppm); s = Singulett, m = Multiplett, bs = verbreitertes Singulett, kb = komplexer Bereich.

¹³C-NMR-Spektroskopie: Die Messungen erfolgten auf einem Bruker AV400 (100 MHz); die Kalibrierung der chemischen Verschiebungen erfolgte relativ zum Lösungsmittelsignal (CDCl₃, δ = 77,16 ppm); HMBC: Hetero multiple bond correlation.

Der Gehalt an Polyoxymethylengruppen, Polypropylenoxidgruppen und Polyethylenoxidgruppen in der Polyolkomponente wurde mit Hilfe von ¹H-NMR Spektroskopie bestimmt. Die relativen Gehalte der einzelnen Inkremente wurden dabei über Integration der charakteristischen Protonensignale bestimmt. Diese wurden auch zur Quantifizierung von Umsätzen herangezogen. Charakteristische Signale der hergestellten Verbindungen sind dabei:
¹H NMR (300 MHz, CDCl₃) δ = 1.14 (m, 3H, Methylgruppen PO); 3.20-3.55 (m, 3H, Ethylengruppen PO); 3.73 (s, 4H, Ethylengruppen EO); 4.70-4.90 (m, 2H, Methylengruppen Formaldehyd FA) ppm.
¹³C NMR (75 MHz, CDCl₃) δ = 17.4 (Methylgruppen PO); 66.9-67.5 (Ethylengruppen PO und EO); 75.5 (Ethylengruppen PO); 73.0 (Ethylengruppen PO und EO); 88.0-95.5 (Methylengruppen Formaldehyd FA); 154.8 (Carbonat) ppm.

### Katalysatorsysnthese

### Synthese des erfindungsgemäß einsetzbaren Katalysators C-1

Zur Synthese von Katalysator C-**1** wurden 1,182 g des 1,5-Naphtalindisulfonsäure-dinatriumsalz (3,556 mmol; 1,0 Eq.) unter inerten Bedingungen in 15 ml absolutem Dichlormethan suspendiert. Unter starkem Rühren wurden 0,19 ml 98 %-ige Schwefelsäure (3,556 mmol; 1,0 Eq.) zugesetzt. Nach zehnminütigem Rühren wurde das Lösemittel im Vakuum entfernt und der Feststoff über 4 Stunden im Hochvakuum getrocknet.

Der Einsatz des erhaltenen Katalysators in weiteren Reaktionen erfolgte ohne weitere Aufreinigung.
D=1,0

Alternativ kann der Katalysator C-**1** auch direkt in Anwesenheit des Starters (BH) in analoger Weise mit und ohne Dichlormethan hergestellt werden.

### Synthese des erfindungsgemäß einsetzbaren Katalysators C-2

Zur Synthese von Katalysator C-**2** wurden 3,709 g des 1,3-Benzoldisulfonsäure-dinatriumsalz (13,158 mmol; 1,0 Eq.) unter inerten Bedingungen in 30 ml absolutem Dichlormethan suspendiert. Unter starkem Rühren wurden 0,70 ml, 98 %-ige Schwefelsäure (13,158 mmol; 1,0 Eq.) zugesetzt. Nach zehnminütigem Rühren wurde das Lösemittel im Vakuum entfernt und der Feststoff über 4 Stunden im Hochvakuum getrocknet.

Der Einsatz des erhaltenen Katalysators in weiteren Reaktionen erfolgte ohne weitere Aufreinigung.
D=1,0

Alternativ kann der Katalysator C-**2** auch direkt in Anwesenheit des Starters (BH) in analoger Weise mit und ohne Dichlormethan hergestellt werden.

### Synthese des erfindungsgemäß einsetzbaren Katalysators C-3

Zur Synthese von Katalysator C-**3** wurden 2,000 g des 1,5-Naphtalindisulfonsäure-dinatriumsalz (6,019 mmol; 1,0 Eq.) unter inerten Bedingungen in 15 ml absolutem Dichlormethan suspendiert. Unter starkem Rühren wurden 0,898 ml Trifluoromethansulfonsäure (1,535 g; 10,233 mmol; 1,7 Eq.) zugesetzt. Nach zehnminütigem Rühren wurde das Lösemittel im Vakuum entfernt.

Der Einsatz des erhaltenen Katalysators C-3 in weiteren Reaktionen erfolgte ohne weitere Aufreinigung.

### D=1,7

Alternativ kann der Katalysator C-**3** auch direkt in Anwesenheit des Starters (BH) in analoger Weise mit und ohne Dichlormethan hergestellt werden.

### Synthese des erfindungsgemäß einsetzbaren Katalysators C-4

In analoger Ausführungsform zur Darstellung von Katalysator C-3 wurde der Katalysator C-**4** mit einem Protonierungsgrad D von D = 1,3 hergestellt. Dabei wurde die Menge der Trifluoromethansulfonsäure entsprechend dem Protonierungsgrad D = 1,3 eingesetzt.
D = 1,3

Alternativ kann der Katalysator C-**4** auch direkt in Anwesenheit des Starters (BH) in analoger Weise mit und ohne Dichlormethan hergestellt werden.

### Synthese des erfindungsgemäß einsetzbaren Katalysators C-5

1,171 g (3.525 mmol) 2,6-Naphtalindisulfonsäuredinatriumsalz wurden in absolutem Dichloromethan suspendiert und mit mit 0,41 ml (4,700 mmol; 0,705 g) Triflourmethansulfonsäure versetzt. Die Suspension wurde über 20 Minuten gerührt und anschließend im Vakuum eingeengt.

Der Einsatz des erhaltenen Katalysators C-5 in weiteren Reaktionen erfolgte ohne weitere Aufreinigung.
D=1,3

Alternativ kann der Katalysator C-**5** auch direkt in Anwesenheit des Starters (BH) in analoger Weise mit und ohne Dichlormethan hergestellt werden.

### Synthese des erfindungsgemäß einsetzbaren Katalysators C-6

In analoger Ausführungsform zur Darstellung von Katalysator C-**5** wurde der Katalysator C-**6** mit einem Protonierungsgrad D von D = 1,7 hergestellt. Dabei wurde die Menge der Trifluoromethansulfonsäure entsprechend dem Protonierungsgrad D = 1,7 eingesetzt.
D = 1,7

Alternativ kann der Katalysator C-**6** auch direkt in Anwesenheit des Starters (BH) in analoger Weise mit und ohne Dichlormethan hergestellt werden.

### Synthese des erfindungsgemäß einsetzbaren Katalysators C-7

Zur Synthese von Katalysator C-**7** wurden 32 mg Fumarsäure (0,280 mmol; 1 Äq) in 0,47 ml einer Lösung aus Tetrabutylammoniummethoxid (0,280 mmol; 20% in Methanol; 1 Äq) aufgelöst. Anschließend wurde die erhaltene Mischung zur Trockene eingeengt.

Der Einsatz des erhaltenen Katalysators C-7 in weiteren Reaktionen erfolgte ohne weitere Aufreinigung.
D=1,0

### Synthese des erfindungsgemäß einsetzbaren Katalysators C-8

In analoger Ausführungsform zur Darstellung von Katalysator C-**7** wurde der Katalysator C-**8** hergestellt wobei 1,5-Naphthalindisulfonsäure anstelle von Fumarsäure als 2-protonige Säure eingesetzt wurde. Der Protonierungsgrad des resultierenden Katalysators C-**8** beträgt D = 1,0.

**Tabelle 1: Herstellung der organischen, n-protonigen Brönsted-Säure(C) als Katalysator**

| Kataly sator | Schritt α | | Schritt β | | D | n |
|---|---|---|---|---|---|---|
| | organische, n-protonige Bronsted-Säure | Base (EH) | Salz der organische, n-protonige Bronsted-Säuren | Säure (E') | | |
| C-1 | | | 1,5-Na2-NDS | H2SO4 | 1,0 | 2 |
| C-2 | | | 1,3-Na2-BDS | H2SO4 | 1,0 | 2 |
| C-3 | | | 1,5-Na2-NDS | CF3SO3H | 1,7 | 2 |
| C-4 | | | 1,5-Na2-NDS | CF3SO3H | 1,3 | 2 |
| C-5 | | | 2,6-Na2-NDS | CF3SO3H | 1,3 | 2 |
| C-6 | | | 2,6-Na2-NDS | CF3SO3H | 1,7 | 2 |
| C-7 | Fumarsäure | (n-Bu)₄N (OMe) | | | 1,0 | 2 |
| C-8 | 1,5-NDS | (n-Bu)₄N (OMe) | | | 1,0 | 2 |

### Aktivitätstests

### Beispiel 1: Umsetzung von Styroloxid mit pFA in Gegenwart von Katalysator C-1

In einem inertisierten Schlenck-Kolben mit Magnetrührer wurden 12,0 g fein gepulverter Paraformaldehyd (pFA, M=450 g/mol; 27,500 mmol; 1,00 Äq) in 25 ml absolutem 1,3-Dioxolan suspendiert. Der Ansatz wurde auf 50°C erwärmt und 119 mg des Katalysators C-1 (0,275 mmol; 0,01 Äq) wurden unter Rühren zugesetzt. Über einen Zeitraum von 72 Stunden wurden 38 ml Styroloxid (330,000 mmol; 12 Äq) zugetropft, wobei sich eine stabile weiße Lösung gebildet hatte. Die Reaktionskontrolle erfolgte mittels NMR. Hierzu wurde das Rohprodukt in Dichlormethan aufgenommen, von ungelösten Bestandteilen abfiltriert und im Vakuum eingeengt.

Der Umsatz von Styroloxid betrug 100%.

Das zahlenmittlere Molekulargewicht Mₙ betrug 1300 g/mol. Die per GPC ermittelte Molekulargewichtsverteilung war monomodal.

Der Polydispersitätsindex betrug 1,99.

### Beispiel 2: Umsetzung von Styroloxid mit pFA in Gegenwart von Katalysator C-1

In einem inertisierten Schlenck-Kolben mit Magnetrührer wurden 44,0 g fein gepulverter pFA (M=450 g/mol; 110,000 mmol; 1,00 Äq) in 25 ml absolutem 1,3-Dioxolan suspendiert. Der Ansatz wurde auf 50°C erwärmt und 474 mg des Katalysators C-**1** (1,100 mmol; 0,01 Äq) wurden unter Rühren zugesetzt. Über einen Zeitraum von 48 Stunden wurden 151 ml Styroloxid (132,000 mmol; 12 Äq) zugetropft, wobei sich eine stabile weiße Lösung gebildet hatte. Die Reaktionskontrolle erfolgte mittels NMR. Hierzu wurde das Rohprodukt in Dichlormethan aufgenommen, von ungelösten Bestandteilen abfiltriert und im Vakuum eingeengt.

Der Umsatz von Styroloxid betrug 100%.

Das zahlenmittlere Molekulargewicht Mₙ betrug 1758 g/mol.

Die per GPC ermittelte Molekulargewichtsverteilung war monomodal.

Der Polydispersitätsindex betrug 1,90.

### Beispiel 3: (Vergleichsbeispiel) Umsetzung von Styroloxid mit PEG in Gegenwart von Natriumhydrogensulfat als Katalysator

In einem inertisierten Schlenk-Kolben mit Magnetrührer wurden 550 mg PEG (M=1000 g/mol; 0,550 mmol; 1,00 Äq) auf 50 °C erwärmt und 1 mg Natriumhydrogensulfat (0,006 mmol; 0,01 Äq) wurden unter Rühren zugesetzt. Anschließend wurden 0,52 ml Styroloxid zugegeben (4,400 mmol; 8,00 Äq) und die Reaktionsmischung über drei Stunden auf 50 °C erhitzt.

Das Reaktionsprodukt wurde ohne weitere Aufreinigung mittels NMR-Spektroskopie analysiert.

Es wurde kein Umsatz des eingesetzten Styroloxids beobachtet.

### Beispiel 4: Umsetzung von Propylenoxid mit PEG in Gegenwart von Katalysator C-5

7.5 mg 2,6-Naphtalindisulfonsäure-dinatriumsalz (0,021 mmol, 0,015 Äq) wurden in einem Ofen-getrockneten Druck resistenten Reaktions-Vial mit 1375 mg PEG (M=1000 g/mol; 1,375 mmol; 1,00 Äq) gemischt. 4 mg Trifluoromethansulfonsäure (0,028 mmol, 0,02 Äq) wurden zugesetzt und das Gemisch bei 60°C gründlich durchmischt. Bei Raumtemperatur wurden 0,77 ml Propylenoxid (11,000 mmol, 8,0 Äq) zugegeben, das Reaktionsgefäß verschlossen und über 1.5 Stunden bei einer Temperatur von 60°C gerührt.

Die weitere Analytik wurde ohne Aufarbeitung der Reaktionsmischung durchgeführt.

Der Umsatz von Propylenoxid betrug 100%.

Mₙ des Produkts betrug 1343 g/mol.

### Beispiel 5: Umsetzung von Propylenoxid mit pFA in Gegenwart von Katalysator C-4

7.5 mg 1,5-Naphtalindisulfonsäure-dinatriumsalz (0,021 mmol; 0,015 Äq) wurden in einem Ofen-getrockneten Druck resistenten Reaktions-Vial mit 550 mg fein gepulverten pFA (M=450 g/mol; 1,375 mmol; 1,00 Äq) gemischt. 4 mg Trifluoromethansulfonsäure (0,028 mmol; 0,02 Äq) wurden zugesetzt und das Gemisch gründlich durchmischt. Von einer Gesamtmenge von 0,77 ml Propylenoxid (11,000 mmol; 8.0 Äq) wurden 0,3 ml zugegeben und gründlich verrührt. Die Zugabe von Propylenoxid wurde anschließend vervollständigt, das Reaktionsgefäß verschlossen und über sechs Stunden bei einer Temperatur von 70°C gerührt.

Die weitere Analytik wurde ohne Aufarbeitung der Reaktionsmischung durchgeführt.

Der Umsatz von Propylenoxid betrug 100%.

Mₙ des Produkts betrug 730 g/mol.

### Beispiel 6: Umsetzung von Propylenoxid mit pFA in Gegenwart von Katalysator C-5

7.5 mg 2,6-Naphtalindisulfonsäure-dinatriumsalz (0,021 mmol; 0,015 Äq) wurden in einem Ofen-getrockneten Druck resistenten Reaktions-Vial mit 550 mg fein gepulverten pFA (M=450 g/mol; 1,375 mmol; 1,00 Äq) gemischt. 4 mg Trifluoromethansulfonsäure (0,028 mmol; 0,02 Äq) wurden zugesetzt und das Gemisch gründlich durchmischt. Von einer Gesamtmenge von 0,77 ml Propylenoxid (11,000 mmol; 8.0 Äq) wurden 0,3 ml zugegeben und gründlich verrührt. Die Zugabe von Propylenoxid wurde anschließend vervollständigt, das Reaktionsgefäß verschlossen und über sechs Stunden bei einer Temperatur von 70°C gerührt.

Die weitere Analytik wurde ohne Aufarbeitung der Reaktionsmischung durchgeführt.

Der Umsatz von Propylenoxid betrug 100%.

Mₙ des Produkts betrug 738 g/mol.

### Beispiel 7: Umsetzung von Allylglycidylether mit PEG in Gegenwart von Katalysator C-6

Unter Inertgas wurden 2,4 mg des Katalysators 6 (0,004 mmol; 0,8 mol%) in einem Ofen-getrockneten Schlenck-Rohr mit 500 mg PEG (M=1000 g/mol; 0,500 mmol; 1,00 Äq)gemischt. Bei Raumtemperatur wurden 0,24 ml Allylglycidylether (2,000 mmol; 4,0 Äq) zugegeben, das Reaktionsgefäß verschlossen und über 8 Stunden bei einer Temperatur von 60°C gerührt.

Die weitere Analytik wurde ohne Aufarbeitung der Reaktionsmischung durchgeführt.

Der Umsatz von Allylglycidylether betrug 82 %.

Das zahlenmittlere Molekulargewicht Mₙ betrug 1634 g/mol.

Die per GPC ermittelte Molekulargewichtsverteilung war im polymeren Bereich monomodal.

Der Polydispersitätsindex betrug 1,8.

### Beispiel 8: Umsetzung von Ethylenoxid mit PET in Gegenwart von Katalysator C-5

1,171 g (3.525 mmol) 2,6-Naphtalindisulfonsäuredinatriumsalz wurden in absolutem Dichloromethan suspendiert und mit 0,41 ml (4,700 mmol; 0,705 g) Triflourmethansulfonsäure versetzt. Die Suspension wurde über 10 Minuten gerührt und anschließend im Vakuum eingeengt. Anschließend wurde der erhaltene weiße Feststoff in 10g PET (0,023 mol) aufgenommen. Die so erhaltene Suspension wurde zusammen mit 91,5 g PET (0,212 mol) in einen 2 L Hochdruckreaktor überführt. Der Inhalt wurde in drei Zyklen mit Stickstoff (1 bar) beaufschlagt und anschließend evakuiert um Luftrückstände zu entfernen. Der Reaktor wurde bei einem Stickstoff-Druck von 45 bar auf 45 °C erwärmt und mit 200 U/min gerührt. Ethylenoxid 142,9 g (0,94 mol; 8 Äq) wurde mit einer Förderrate von 2 ml/min zugegeben und die Reaktionstemperatur wurde mit 10°C/15 min erhöht, bis eine Innentemperatur von 105°C erreicht war (oberhalb von 90°C wurde die exotherme Reaktion beobachtet). Nach Abkühlen auf 60°C wurde die Ethylenoxid-Konzentration in der Gasphase bestimmt (unterhalb von 5 ppm) und der Reaktor wurde entspannt.

Der Umsatz von Ethylenoxid betrug 100 %. Der Anteil an Nebenkomponenten konnte mit 0,6 Gew.-% ermittelt werden.

Das zahlenmittlere Molekulargewicht Mₙ betrug 740 g/mol.

Die per GPC ermittelte Molekulargewichtsverteilung war im gesamten Bereich monomodal.

Der Polydispersitätsindex betrug 1,2.

### Beispiel 9: Umsetzung von Ethylenoxid mit pFA in Gegenwart von Katalysator C-5

1,220 g (3.673 mmol) 2,6-Naphtalindisulfonsäuredinatriumsalz wurden in absolutem Dichloromethan suspendiert und mit 0,72 g (4,797 mmol) Triflourmethansulfonsäure versetzt. Die Suspension wurde über 10 Minuten gerührt und anschließend im Vakuum eingeengt. Anschließend wurde der erhaltene weiße Feststoff in 108 g cPC aufgenommen. Die so erhaltene Suspension wurde überführt. Anschließend werden 105,8 g pFA (M=450 g/mol, 0,235 mol) addiert. Der Inhalt wurde in drei Zyklen mit Stickstoff (1 bar) beaufschlagt und anschließend evakuiert um Luftrückstände zu entfernen. Der Reaktor wurde bei einem Stickstoff-Druck von 45 bar auf 60 °C erwärmt und mit 200 U/min gerührt. Ethylenoxid 142,8 g (0,94 mol, 8 Äq) wurde mit einer Förderrate von 2 ml/min zugegeben und die Reaktionstemperatur wurde mit 10°C/15 min erhöht, bis eine Innentemperatur von 105°C erreicht war (oberhalb von 90°C wurde die exotherme Reaktion beobachtet). Nach Abkühlen auf 60°C wurde die Ethylenoxid-Konzentration in der Gasphase bestimmt (unterhalb von 5 ppm) und der Reaktor wurde entspannt.

Der Umsatz von Ethylenoxid betrug 100 %. Der Anteil an Nebenkomponenten konnte mit 8,6 Gew.-% ermittelt werden.

Das zahlenmittlere Molekulargewicht Mₙ betrug 1409 g/mol.

Die per GPC ermittelte Molekulargewichtsverteilung war im gesamten Bereich monomodal.

Der Polydispersitätsindex betrug 1,9.

### Beispiel 10: Umsetzung von 1,3-Dioxolan mit pFA in Gegenwart von Katalysator C-2

12 g feingepulverter pFA (0,030 mol; 1,0 Äq) wurden unter inerten Bedingungen in 12 ml absolutem 1,3-Dioxolan suspendiert. Es wurde auf 65 °C erhitzt und der Katalysator **2** (342 mg; 0,009 mol; 0,03 Äq) zugegeben. Die Reaktionsmischung wurde über 7,5 Stunden gerührt. Das Produktgemisch wurde in 40 ml Dichlormethan aufgenommen, von ungelösten Bestandteilen abfiltriert und im Vakuum eingeengt.
Auswaage: 10,2 g

Das zahlenmittlere Molekulargewicht Mₙ betrug 1953 g/mol.

Die per GPC ermittelte Molekulargewichtsverteilung war monomodal.

Der Polydispersitätsindex betrug 1,8.

### Beispiel 11: Umsetzung von Propylenoxid mit PC in Gegenwart von Katalysator C-5

7,5 mg 2,6-Naphtalindisulfonsäure-dinatriumsalz (0,021 mmol; 0,015 Äq) wurden in einem Ofen-getrockneten Druck resistenten Reaktions-Vial mit 4 mg Trifluoromethansulfonsäure (0,028 mmol; 0,02 Äq) gemischt. 1375 mg PC (M=1000 g/mol; 1,375 mmol; 1,00 Äq) wurden in 0,77 ml Propylenoxid (11,000 mmol, 8,0 Äq) gelöst zugegeben und gründlich verrührt. Das Reaktionsgefäß wurde verschlossen und über fünf Stunden bei einer Temperatur von 75°C gerührt.

Die weitere Analytik wurde ohne Aufarbeitung der Reaktionsmischung durchgeführt.

Es wurde ein vollständiger Umsatz des eingesetzten Propylenoxids unter vernachlässigbarer Bildung von cyclischem Propylencarbonat beobachtet. Die Neubildung von cyclischem Propylencarbonat wurde mittels Protonenresonanzspektroskopie ermittelt und betrug 2,7% der alternierenden Polycarbonatgruppen.

Das zahlenmittlere Molekulargewicht Mₙ betrug 1381 g/mol.

Die per GPC ermittelte Molekulargewichtsverteilung war monomodal.

Der Polydispersitätsindex betrug 1,3.

### Beispiel 12: (Vergleichsbeispiel) Umsetzung von Propylenoxid mit PC in Gegenwart von 1,8-Diazabicyclo[5.4.0]undec-7-en (DBU) als Katalysator

3 mg 1,8-Diazabicyclo[5.4.0]undec-7-en (0,015 mmol; 0,015 Äq) wurden in einem Ofen-getrockneten Druck resistenten Reaktions-Vial vorgelegt. 1375 mg PC (M=1000 g/mol; 1,375 mmol; 1,00 Äq.) wurden in 0,77 ml Propylenoxid (11,000 mmol, 8,0 Äq) gelöst zugegeben und gründlich verrührt. Das Reaktionsgefäß wurde verschlossen und über fünf Stunden bei einer Temperatur von 75°C gerührt.

Das Reaktionsprodukt wurde ohne weitere Aufreinigung mittels NMR-Spektroskopie analysiert.

Es wurde ein vollständiger Abbau des Polymers zu cyclischem Propylencarbonat beobachtet. Es konnte kein Umsatz von eingesetztem Propylenoxid zu polymeren Strukturen beobachtet werden.

### Beispiel 13: Umsetzung von Styroloxid mit PEG in Gegenwart von Katalysator C-7

10 mg Katalysator C-**7** Tetrabutylammoniumhydrogenfumarat (0,028 mmol; 0,100 Äq) wurden mit 275 mg PEG (M=1000 g/mol; 0,275 mmol; 1,000 Äq) gemischt. Die Mischung wurde auf 70°C erhitzt und 0,13 ml Styroloxid (1,100 mmol, 4,0 Äq) wurden zugegeben. Der Ansatz wurde über 10 Stunden bei 70°C gerührt.

Das Reaktionsprodukt wurde ohne weitere Aufreinigung mittels NMR-Spektroskopie analysiert.

Der Umsatz an eingesetztem Styroloxid betrug 28%.

Aus der NMR-Analytik wurde ein Mₙ von 1135 g/mol berechnet.

### Beispiel 14 (Vergleichsbeispiel): Umsetzung von Propylenoxid mit PEG in Gegenwart von Trifluoromethansulfonsäure als Katalysator

In einem Ofen-getrockneten Druck resistenten Reaktions-Vial mit 1375 mg PEG (M=1000 g/mol; 1,375 mmol; 1,00 Äq) vorgelegt. 4 mg Trifluoromethansulfonsäure (CF₃SO₃H, 0,028 mmol, 0,02 Äq) wurden zugesetzt und das Gemisch bei 60°C gründlich durchmischt. Bei Raumtemperatur wurden 0,77 ml Propylenoxid (11,000 mmol, 8,0 Äq) zugegeben, das Reaktionsgefäß verschlossen und über 1.5 Stunden bei einer Temperatur von 60°C gerührt.

Die weitere Analytik wurde ohne Aufarbeitung der Reaktionsmischung durchgeführt.

Der Umsatz von Propylenoxid betrug 100%.

Die per GPC ermittelte Molekulargewichtsverteilung war im polymeren Bereich multimodal und wies hohe Anteile niedermolekularer Verbindungen auf. Mₙ betrug 1292 g/mol.

### Beispiel 15 (Vergleichsbeispiel): Umsetzung von Propylenoxid mit PC in Gegenwart von Trifluoromethansulfonsäure als Katalysator

In einem Ofen-getrockneten Druck resistenten Reaktions-Vial wurden 4 mg Trifluoromethansulfonsäure (0,028 mmol; 0,02 Äq) vorgelegt. 1375 mg PC (M=1000 g/mol; 1,375 mmol; 1,00 Äq) wurden in 0,77 ml Propylenoxid (11,000 mmol, 8,0 Äq) gelöst zugegeben und gründlich verrührt. Das Reaktionsgefäß wurde verschlossen und über fünf Stunden bei einer Temperatur von 75°C gerührt.

Die weitere Analytik wurde ohne Aufarbeitung der Reaktionsmischung durchgeführt.

Es wurde ein vollständiger Umsatz des eingesetzten Propylenoxids unter starker Bildung von cyclischem Propylencarbonat beobachtet. Die Neubildung von cyclischem Propylencarbonat wurde mittels NMR ermittelt und betrug 39% der alternierenden Polyethercarbonatgruppen.

Das zahlenmittlere Molekulargewicht Mₙ betrug 808 g/mol.

Die per GPC ermittelte Molekulargewichtsverteilung wies hohe Anteile niedermolekularer Verbindungen auf.

Der Polydispersitätsindex betrug 1,6.

### Beispiel 16 (Vergleichsbeispiel): Umsetzung von Propylenoxid mit pFA in Gegenwart von Triflourmethansulfonsäure als Katalysator

In einem Ofen-getrockneten Druck resistenten Reaktions-Vial wurden 550 mg fein gepulverter pFA (M=450 g/mol; 1,375 mmol; 1,00 Äq) vorgelegt. 4 mg Trifluoromethansulfonsäure (0,028 mmol; 0,02 Äq) wurden zugesetzt und das Gemisch gründlich durchmischt. Von einer Gesamtmenge von 0,77 ml Propylenoxid (11,000 mmol; 8.0 Äq) wurden 0,3 ml zugegeben und gründlich verrührt. Die Zugabe von Propylenoxid wurde anschließend vervollständigt, das Reaktionsgefäß verschlossen und über sechs Stunden bei einer Temperatur von 70°C gerührt.

Die weitere Analytik wurde ohne Aufarbeitung der Reaktionsmischung durchgeführt.

Der Umsatz von Propylenoxid betrug 100%.

Die per GPC ermittelte Molekulargewichtsverteilung war im polymeren Bereich multimodal und Mₙ betrug 476g/mol.

### Beispiel 17 (Vergleichsbeispiel): Umsetzung von Propylenoxid mit PEG in Gegenwart von 2,6-Naphtalindisulfonsäure-dinatriumsalz als Katalysator

7.5 mg 2,6-Naphtalindisulfonsäure-dinatriumsalz (0,021 mmol, 0,015 Äq) wurden in einem Ofen-getrockneten Druck resistenten Reaktions-Vial mit 1375 mg PEG (M=1000 g/mol; 1,375 mmol; 1,00 Äq) gemischt und das Gemisch bei 60°C gründlich durchmischt. Bei Raumtemperatur wurden 0,77 ml Propylenoxid (11,000 mmol, 8,0 Äq) zugegeben, das Reaktionsgefäß verschlossen und über 1.5 Stunden bei einer Temperatur von 60°C gerührt.

Die weitere Analytik wurde ohne Aufarbeitung der Reaktionsmischung durchgeführt.

Der Umsatz von Propylenoxid betrug 0%.

### Beispiel 18 (Vergleichsbeispiel): Umsetzung von Propylenoxid mit PEG in Gegenwart von 1,5-Naphtalindisulfonsäure-dinatriumsalz als Katalysator

7.5 mg 1,5-Naphtalindisulfonsäure-dinatriumsalz (0,021 mmol, 0,015 Äq) wurden in einem Ofen-getrockneten Druck resistenten Reaktions-Vial mit 1375 mg PEG (M=1000 g/mol; 1,375 mmol; 1,00 Äq) gemischt und das Gemisch bei 60°C gründlich durchmischt. Bei Raumtemperatur wurden 0,77 ml Propylenoxid (11,000 mmol, 8,0 Äq) zugegeben, das Reaktionsgefäß verschlossen und über 1.5 Stunden bei einer Temperatur von 60°C gerührt.

Die weitere Analytik wurde ohne Aufarbeitung der Reaktionsmischung durchgeführt.

Der Umsatz von Propylenoxid betrug 0%.

### Beispiel 19 Umsetzung von Styroloxid mit PEG in Gegenwart von Katalysator C-8

In einem inertisierten Schlenk-Kolben mit Magnetrührer wurden 1375 mg PEG (M=1000 g/mol; 1,375 mmol; 1,00 Äq) auf 50 °C erwärmt und 12 mg Katalysator 8 (0,020 mmol; 0,01 Äq) wurden unter Rühren zugesetzt. Anschließend wurden 1,25 ml Styroloxid zugegeben (11,000 mmol; 8,00 Äq) und die Reaktionsmischung über zwei Stunden auf 50 °C erhitzt.

Das Reaktionsprodukt wurde ohne weitere Aufreinigung mittels NMR-Spektroskopie analysiert.

Es wurde ein vollständiger Umsatz des eingesetzten Styroloxids mittels NMR beobachtet.

Mn des Produkts betrug 2209g/mol.

### Beispiel 20 (Vergleichsbeispiel): Umsetzung von Propylenoxid mit PEG in Gegenwart von Schwefelsäure als Katalysator

In einem Ofen-getrockneten Druck resistenten Reaktions-Vial wurden 3 mg Schwefelsäure, 98%, (0,028 mmol; 0,02 Äq) vorgelegt. 1375 mg PEG (M=1000 g/mol; 1,375 mmol; 1,00 Äq) wurden zugesetzt und bei Raumtemperatur wurden 0,77 ml Propylenoxid (11,000 mmol, 8,0 Äq) zugegeben, das Reaktionsgefäß verschlossen und über 1.5 Stunden bei einer Temperatur von 60°C gerührt.

Die weitere Analytik wurde ohne Aufarbeitung der Reaktionsmischung durchgeführt.

Der Umsatz von Propylenoxid betrug 8%.

### Beispiel 21 (Vergleichsbeispiel): Umsetzung von Propylenoxid mit PEG in Gegenwart von Natriumhydrogensulfat als Katalysator

In einem Ofen-getrockneten Druck resistenten Reaktions-Vial wurden 4 mg Natriumhydrogensulfat (NaHSO₄, 0,028 mmol; 0,02 Äq) vorgelegt. 1375 mg PEG (M=1000 g/mol; 1,375 mmol; 1,00 Äq) wurden zugesetzt und bei Raumtemperatur wurden 0,77 ml Propylenoxid (11,000 mmol, 8,0 Äq) zugegeben, das Reaktionsgefäß verschlossen und über 1.5 Stunden bei einer Temperatur von 60°C gerührt.

Die weitere Analytik wurde ohne Aufarbeitung der Reaktionsmischung durchgeführt.

Der Umsatz von Propylenoxid betrug 0%.

### Beispiel 22 (Vergleichsbeispiel): Umsetzung von Propylenoxid mit PEG in Gegenwart von Natriumtriflat als Katalysator

In einem Ofen-getrockneten Druck resistenten Reaktions-Vial wurden 5 mg Natriumtriflat (NaOTf, 0,028 mmol; 0,02 Äq) vorgelegt. 1375 mg PEG (M=1000 g/mol; 1,375 mmol; 1,00 Äq) wurden zugesetzt und bei Raumtemperatur wurden 0,77 ml Propylenoxid (11,000 mmol, 8,0 Äq) zugegeben, das Reaktionsgefäß verschlossen und über 1.5 Stunden bei einer Temperatur von 60°C gerührt.

Die weitere Analytik wurde ohne Aufarbeitung der Reaktionsmischung durchgeführt.

Der Umsatz von Propylenoxid betrug 0%.

### Beispiel 23 (Vergleichsbeispiel): Umsetzung von Propylenoxid mit PEG in Gegenwart von Natriumhydrogensulfat-Natriumtriflat-Gemisch als Katalysator

In einem Ofen-getrockneten Druck resistenten Reaktions-Vial wurden 4 mg Natriumhydrogensulfat und 5mg Natriumtriflat (0,028 mmol; 0,02 Äq) vorgelegt. 1375 mg PEG (M=1000 g/mol; 1,375 mmol; 1,00 Äq) wurden zugesetzt und bei Raumtemperatur wurden 0,77 ml Propylenoxid (11,000 mmol, 8,0 Äq) zugegeben, das Reaktionsgefäß verschlossen und über 1.5 Stunden bei einer Temperatur von 60°C gerührt.

Die weitere Analytik wurde ohne Aufarbeitung der Reaktionsmischung durchgeführt.

Der Umsatz von Propylenoxid betrug 0%.

### Beispiel 24: Umsetzung von Propylenoxid mit PC in Gegenwart von Katalysator C-5

150 mg 2,6-Naphtalindisulfonsäure-dinatriumsalz (0,450 mmol; 0,015 Äq) wurden in einem Ofen-getrockneten Glaskolben mit 2 ml absolutiertem Dichloromethan suspendiert. Unter Rühren wurden 90 mg Trifluoromethansulfonsäure (0,600 mmol, 0,020 Äq) zugegeben. Nach dreißigminütigem Rühren wurde das Lösemittel im Vakuum entfernt. In einen Reaktor aus Edelstahl mit Einlage aus Teflon wurden 30,000 g PC (M = 1000 g/mol, 30,000 mmol, 1,00 Äq) des PC in 25 ml absolutem Tetrahydrofuran gelöst. Anschließend wurde der frisch hergestellte Katalysator zugesetzt und der Reaktor verschlossen. Bei Raumtemperatur wurden 7,000 g Propylenoxid (120,000 mmol, 4,00 Äq) zugegeben und unter Rühren auf 75°C erhitzt. Nach zweistündiger Reaktionszeit wurden weitere 7,000 g Propylenoxid zugesetzt, so dass eine Gesamtmenge von 14,000 g (240,000 mmol, 8,00 Äq) dosiert wurde. Nach einer gesamten Reaktionszeit von 5 Stunden und 30 Minuten wurde auf Raumtemperatur gekühlt und der Inhalt des Reaktors entnommen.

Erhalten wurden 58,0g einer klaren Flüssigkeit, die ohne weitere Aufreinigung analysiert wurde.

Der Umsatz von Propylenoxid betrug 100%

Der auf Grund von Protonen-Resonanzspektroskopie ermittelte Gehalt an cyclischem Propylencarbonat betrug 1,2 Gew.-% bezogen auf eingesetztes Polyethercarbonat (das verwendete Ausgangsmaterial enthält bereits 3,0 Gew.-% cyclisches Propylencarbonat). Der Anteil gebildeten cyclischen Ethern betrug 8,2 Gew.-% (bezogen auf das Reaktionsgemisch).

Das zahlenmittlere Molekulargewicht Mₙ betrug 1633 g/mol.

Der Polydispersitätsindex betrug 1,3.

### Beispiel 25 (Vergleichsbeispiel): Umsetzung von Propylenoxid mit PC in Gegenwart von Trifloumethansulfonsäure als Katalysator

In einen Reaktor aus Edelstahl mit Einlage aus Teflon wurden 30,000 g PC (M = 1000 g/mol, 30,000 mmol, 1,00 Äq) in 25 ml absolutem Tetrahydrofuran gelöst. Anschließend wurden 150 mg Triflourmethansulfonsäure (0,600 mmol, 0,02 Äq) zugesetzt und der Reaktor verschlossen. Bei Raumtemperatur wurden 7,000 g Propylenoxid (120,000 mmol, 4,00 Äq) zugegeben und unter Rühren auf 75°C erhitzt. Nach zweistündiger Reaktionszeit wurden weitere 7,000 g Propylenoxid zugesetzt, so dass eine Gesamtmenge von 14,000 g (240,000 mmol, 8,00 Äq) dosiert wurde. Nach einer gesamten Reaktionszeit von 5 Stunden und 30 Minuten wurde auf Raumtemperatur gekühlt und der Inhalt des Reaktors entnommen.
Der Umsatz von Propylenoxid betrug 100%

Der auf Grund von Protonen-Resonanzspektroskopie ermittelte Bildung an cyclischem Propylencarbonat betrug 22,3 Gew.-% bezogen auf eingesetzte Polycarbonatpolyols (das verwendete Ausgangsmaterial enthält bereits 3,0 Gew.-% cyclisches Propylencarbonat). Der Anteil gebildeten cyclischen Ethern betrug 13,1 Gew.-% (bezogen auf das Reaktionsgemisch).

Das zahlenmittlere Molekulargewicht Mₙ betrug 1340 g/mol.

Der Polydispersitätsindex betrug 1,4.

### Beispiel 26: Umsetzung von Maleinsäureanhydrid mit Propylenoxid und PET in Gegenwart von Katalysator C-5

300 mg 2,6-Naphtalindisulfonsäure-dinatriumsalz (0,900 mmol; 0,015 Äq) wurden in einem Ofen-getrockneten Glaskolben mit 8 ml absolutiertem Dichloromethan suspendiert. Unter Rühren wurden 180 mg Trifluoromethansulfonsäure (1,200 mmol, 0,020 Äq) zugegeben. Nach dreißigminütigem Rühren wurde das Lösemittel im Vakuum entfernt. In einen Reaktor aus Edelstahl mit Einlage aus Teflon wurden 26,000 g (60,000 mmol, 1,00 Äq) des PET-Polyols mit einem Molekulargewicht von 432 g/mol mit 11,769 g Maleinsäureanhydrid (120,000 mmol; 2,0 Äq) gemischt. Anschließend wurde der frisch hergestellte Katalysator zugesetzt und der Reaktor verschlossen. Bei einer Temperatur von 80 °C wurden portionsweise insgesamt 28,000 g Propylenoxid (480,000 mmol, 8,00 Äq) innerhalb von 3 Stunden zugegeben. Nach kompletter Zugabe wurde die Temperatur auf 100 °C erhöht und das Gemisch für weitere zwei Stunden gerührt. Anschließend wurde auf Raumtemperatur gekühlt und der Inhalt des Reaktors entnommen. Die gelbliche Flüssigkeit wurde mit 40 ml Dichlormethan aufgenommen, über Papierfilter filtriert um ungelöstes Maleinsäureanhydrid zu entfernen und anschließend im Vakuum eingeengt. Erhalten wurden 48,2 g einer klaren hell gelben Flüssigkeit (73%).

Der auf Grund von Protonen-Resonanzspektroskopie des unfiltrierten Rohprodukts ermittelte Umsatz an Propylenoxid war 100%. Der Umsatz an eingesetztem Maleinsäureanhydrid betrug 94%.

Die Konnektivität zwischen eingebauten Maleinsäure-Äquivalenten und Polyether konnte durch 1H-13C-korrelierte Magnetresonanzspektroskopie des gereinigten Produkts gezeigt werden.

Das Molekulargewicht MPeak betrug 1107 g/mol.

Der Polydispersitätsindex betrug 1,3.

### Beispiel 27: Umsetzung von ε-Caprolacton (A-2) mit Propylenoxid (A-1) und PET in Gegenwart von Katalysator C-5

300 mg 2,6-Naphtalindisulfonsäure-dinatriumsalz (0,900 mmol; 0,015 Äq) wurden in einem Ofen-getrockneten Glaskolben mit 8 ml absolutiertem Dichloromethan suspendiert. Unter Rühren wurden 180 mg Trifluoromethansulfonsäure (1,200 mmol, 0,020 Äq) zugegeben. Nach dreißigminütigem Rühren wurde das Lösemittel im Vakuum entfernt. In einen Reaktor aus Edelstahl mit Einlage aus Teflon wurden 26,000 g (60,000 mmol, 1,00 Äq) des PET-Polyols mit einem Molekulargewicht von 432 g/mol mit 13,697 g ε-Caprolacton (120,000 mmol; 2,0 Äq) gemischt. Anschließend wurde der frisch hergestellte Katalysator zugesetzt und der Reaktor verschlossen. Bei einer Temperatur von 80°C wurden portionsweise insgesamt 28,000 g Propylenoxid (480,000 mmol, 8,00 Äq) zugegeben. Nach dreistündigem Rühren bei 80°C und einstündigem Rühren bei 100°C wurde die Reaktion beendigt. Nach Kühlen auf Raumtemperatur wurde der Inhalt des Reaktors entnommen (66,4g; 98%).

Der auf Grund von Protonen-Resonanzspektroskopie des Rohprodukts ermittelte Umsatz an Propylenoxid war 100%. Der Umsatz an eingesetztem ε-Caprolacton betrug ebenfalls 100%.

Das Molekulargewicht MPeak betrug 1349 g/mol.

Der Polydispersitätsindex betrug 1,5.

### Beispiel 28: Umsetzung von β-Butyrolacton (A-2) mit Propylenoxid (A-1) und PET in Gegenwart von Katalysator C-5

300 mg 2,6-Naphtalindisulfonsäure-dinatriumsalz (0,900 mmol; 0,015 Äq) wurden in einem Ofen-getrockneten Glaskolben mit 8 ml absolutiertem Dichloromethan suspendiert. Unter Rühren wurden 180 mg Trifluoromethansulfonsäure (1,200 mmol, 0,020 Äq) zugegeben. Nach dreißigminütigem Rühren wurde das Lösemittel im Vakuum entfernt. In einen Reaktor aus Edelstahl mit Einlage aus Teflon wurden 26,000 g (60,000 mmol, 1,00 Äq) des PET-Polyols mit einem Molekulargewicht von 432 g/mol mit 10,330 g β-Butyrolacton (120,000 mmol; 2,0 Äq) gemischt. Anschließend wurde der frisch hergestellte Katalysator zugesetzt und der Reaktor verschlossen. Bei einer Temperatur von 80°C wurden portionsweise insgesamt 28,000 g Propylenoxid (480,000 mmol, 8,00 Äq) zugegeben. Nach dreistündigem Rühren wurde die Reaktion beendigt, auf Raumtemperatur gekühlt und der Inhalt des Reaktors entnommen. Die gelbliche Flüssigkeit wurde mit 40 ml Dichlormethan aufgenommen, über Papierfilter filtriert und anschließend im Vakuum eingeengt. Erhalten wurden 54.5 g einer klaren hell gelben Flüssigkeit (84,0 %).

Der auf Grund von Protonen-Resonanzspektroskopie des unfiltrierten Rohprodukts ermittelte Umsatz an Propylenoxid war 100%. Der Umsatz an eingesetztem β-Butyrolacton betrug 77%.

Das Molekulargewicht MPeak betrug 1295 g/mol.

Der Polydispersitätsindex betrug 1,3.

### Beispiel 29: Umsetzung von Tetrahydrofuran (A-7) mit Propylenoxid (A-1) und PET in Gegenwart von Katalysator C-5

300 mg 2,6-Naphtalindisulfonsäure-dinatriumsalz (0,900 mmol; 0,015 Äq) wurden in einem Ofen-getrockneten Glaskolben mit 8 ml absolutiertem Dichloromethan suspendiert. Unter Rühren wurden 180 mg Trifluoromethansulfonsäure (1,200 mmol, 0,020 Äq) zugegeben. Nach dreißigminütigem Rühren wurde das Lösemittel im Vakuum entfernt. In einen Reaktor aus Edelstahl mit Einlage aus Teflon wurden 26,000 g (60,000 mmol, 1,00 Äq) des PET-Polyols mit einem Molekulargewicht von 432 g/mol mit 8,654 g Tetrahydrofuran (120,000 mmol; 2,0 Äq) gemischt. Anschließend wurde der frisch hergestellte Katalysator zugesetzt und der Reaktor verschlossen. Bei einer Temperatur von 100°C wurden portionsweise insgesamt 28,000 g Propylenoxid (480,000 mmol, 8,00 Äq) zugegeben. Nach viereinhalbstündigem Rühren wurde die Reaktion beendigt, auf Raumtemperatur gekühlt und der Inhalt des Reaktors entnommen. Die gelbliche Flüssigkeit wurde mit 40 ml Dichlormethan aufgenommen, über Papierfilter filtriert und anschließend im Vakuum eingeengt. Erhalten wurden 48,3 g einer klaren hell gelben Flüssigkeit (77%).

Der auf Grund von Protonen-Resonanzspektroskopie des unfiltrierten Rohprodukts ermittelte Umsatz an Propylenoxid war 100%. Der Umsatz an eingesetztem Tetrahydrofuran betrug 64%.

Die Konnektivität zwischen eingebauten 1,4-Dihydroxybutan-Äquivalenten und Polyether konnte durch 1H-13C-korrelierte Magnetresonanzspektroskopie des gereinigten Produkts gezeigt werden.

Das Molekulargewicht MPeak betrug 1132 g/mol.

Der Polydispersitätsindex betrug 1,4.

### Beispiel 30: Umsetzung von 1,3-Dioxolan (A-4) mit Propylenoxid (A-1) und Paraformaldehyd (pFA) in Gegenwart von Katalysator C-5

187 mg 2,6-Naphtalindisulfonsäure-dinatriumsalz (0,563 mmol; 0,027 Äq) wurden in einem Ofen-getrockneten Glaskolben mit 0,5 ml absolutiertem Dichloromethan suspendiert. Unter Rühren wurden 113 mg Trifluoromethansulfonsäure zugegeben. Nach fünfminütigem Rühren wurde das Lösemittel im Vakuum entfernt und der pulvrige weiße Feststoff mit 15,000 g fein gepulvertem Paraformaldehyd pFA (M=450 g/mol; 33,333 mmol; 1,00 Äq) gemischt. Das erhaltene Pulver wurde in einen Reaktor aus Edelstahl mit Einlage aus Teflon gegeben und dort mit 34 ml absolutiertem 1,3-Dioxolan suspendiert. Anschließend wurde der Reaktor verschlossen und unter Rühren auf 60°C aufgeheizt und Propylenoxid in Portionen von 5 ml zu dosiert. Nachdem über einen Zeitraum von 30 Minuten 10 ml zugesetzt worden waren, wurde die Innentemperatur des Reaktors auf 70°C erhöht. Die Zugabe von Propylenoxid wurde bis zu einer Gesamtmenge von 20 ml (300,000 mmol; 9,0 Äq) fortgesetzt und über einen Zeitraum von 18 Stunden bei 70°C nachgerührt.

Durch Protonenmagnetresonanzspektroskopie der rohen Produktmischung wurde ein vollständiger Umsatz des eingesetzten Propylenoxids festgestellt.

Die erhaltene Lösung wurde in 30 ml Dichloromethan aufgenommen, filtriert und im Vakuum eingeengt. Dabei wurden 42,3 g einer gelblichen Flüssigkeit erhalten.

Der Umsatz von Propylenoxid betrug 100%, Die Ausbeute (inklusive 1,3-Dioxolan als Comonomer) betrug 62%.

Der auf Grund von Protonen-Resonanzspektroskopie ermittelte Gehalt an Formaldehyd betrug 42 Gew. %.

Das zahlenmittlere Molekulargewicht Mn betrug 1652 g/mol.

Der Polydispersitätsindex betrug 1,5.

### Beispiel 31(Vergleichsbeispiel): Umsetzung von Propylenoxid mit PEG in Gegenwart von Perfluorsulfonsäure Membran (Nafion® N117)

31 mg Perfluorsulfonsäure Membran vom Typ Nafion®. N117 mit einer Äquivalentmenge von 0.91-1.11 mmol Sulfonylgruppen pro Gramm (0,028 mmol, 0,020 Äq) wurden in einem Ofen-getrockneten Druck-resistenten Reaktions-Vial mit 1375 mg PEG (M=1000 g/mol; 1,375 mmol; 1,00 Äq) vorgelegt. Bei Raumtemperatur wurden 0,77 ml Propylenoxid (11,000 mmol, 8,0 Äq) zugegeben, das Reaktionsgefäß verschlossen und über 1.5 Stunden bei einer Temperatur von 60°C gerührt.

Die weitere Analytik wurde ohne Aufarbeitung der Reaktionsmischung durchgeführt.

Der Umsatz von Propylenoxid betrug 16%.

**Tabelle 2: Test auf Aktivität von erfindungsgemäßen und vergleichenden Katalysatorsystemen mit Propylenoxid (A-1), PEG (BH), einer Reaktionstemperatur von T=60°C, einer Rektionszeit von t=1,5 h, einem molaren Verhältnis n(A-l)/n(BH) = 8.**

| # | Katalysator | organisch | n | D | X (A-1) [%] | Mₙ [g/mol] |
|---|---|---|---|---|---|---|
| 21 Vgl. | NaHSO4 | Nein | 2 | 1,0 | 0 | - |
| 22 Vgl. | NaOTf | Ja | 1 | 0 | 0 | - |
| 23 Vgl. | NaHSO4/NaOTf | Ja | 3 | 1,0 | 0 | - |
| 14 Vgl. | CF3SO3H | Ja | 1 | 1,0 | 100 | 1292^{a} |
| 18 Vgl. | 1,5-Na2NDS | Ja | 2 | 0 | 0 | - |
| 17 Vgl. | 2,6-Na2NDS | Ja | 2 | 0 | 0 | - |
| 4 | **C-5** | Ja | 2 | 1,3 | 100 | 1343 |
| 20 Vgl. | H2SO4 | Nein | 2 | 2,0 | 8 | - |
| 31 Vgl. | Nafion®. N117 | Ja | - | - | 16 | - |

| | | | | | | |
|---|---|---|---|---|---|---|
| a) Multimodale Molekulargewichtsverteilung. | | | | | | |

**Tabelle 3: Test auf Aktivität von erfindungsgemäßen und vergleichenden Katalysatorsystemen mit Styroloxid (A-1), PEG (BH), einer Reaktionstemperatur von T=50°C, einer Rektionszeit von t=2 h, einem molaren Verhältnis (A-1)/n(BH) = 8.**

| # | Katalysator | organisch | n | D | X (A-1) [%] | Mₙ [g/mol] |
|---|---|---|---|---|---|---|
| 19 | **C-8** | ja | 2 | 1,0 | 100 | 2209 |
| 3 Vgl. | NaHSO₄ | nein | 2 | 1,0 | 0 | - |

**Tabelle 4: Test auf Aktivität des erfindungsgemäßen Katalysatorsystems C-6 mit AGE (A-1), PEG (BH), einer Reaktionstemperatur von T=60°C, einer Rektionszeit von t=2 h, einem molaren Verhältnis (A-1)/n(BH) = 4.**

| # | Katalysator | organisch | n | D | X (A-1) [%] | Mₙ [g/mol] |
|---|---|---|---|---|---|---|
| 7 | **C-6** | ja | 2 | 1,7 | 82 | 1634 |

**Tabelle 5: Test auf Aktivität von erfindungsgemäßen und vergleichenden Katalysatorsystemen mit Propylenoxid (A-1), pFA (BH), einer Reaktionstemperatur von T=70°C, einer Rektionszeit von t=6 h, einem molaren Verhältnis (A-1)/n(BH) = 8.**

| # | Katalysator | organisch | n | D | X (A-1) [%] | Mₙ [g/mol] |
|---|---|---|---|---|---|---|
| 5 | **C-4** | Ja | 2 | 1.3 | 100 | 730 |
| 6 | **C-5** | Ja | 2 | 1.3 | 100 | 738 |
| 16 Vgl. | CF3SO3H | Ja | 1 | 1,0 | 100 | 476^{a} |

| | | | | | | |
|---|---|---|---|---|---|---|
| a) Multimodale Molekulargewichtsverteilung. | | | | | | |

**Tabelle 6: Test auf Aktivität von erfindungsgemäßen Katalysatorsystemen mit Styroloxid (A-1), pFA (BH), einer Reaktionstemperatur von T=50°C, einem molaren Verhältnis (A-1)/n(BH) =12.**

| # | Katalysator | organisch | n | D | X (A-1) [%] | Mₙ [g/mol] |
|---|---|---|---|---|---|---|
| 1 | **C-1** | Ja | 2 | 1,0 | 100 | 1300 |
| 2 | **C-1** | Ja | 2 | 1,0 | 100 | 1758 |

**Tabelle 7: Test auf Aktivität des erfindungsgemäßen Katalysatorsystems mit 1,3-Dioxolan (A-4), pFA (BH), einer Reaktionstemperatur von T=65°C, einem molaren Verhältnis (A-4)/(BH) =5,7.**

| # | Katalysator | organisch | n | D | X (A-4) [%] | Mₙ [g/mol] |
|---|---|---|---|---|---|---|
| 10 | **C-2** | ja | 2 | 1,0 | 37* | 1953 |

| | | | | | | |
|---|---|---|---|---|---|---|
| * 1,3-Dioxolan auch Lösungsmittel | | | | | | |

**Tabelle 8: Test auf Aktivität und Selektivität von erfindungsgemäßen und vergleichenden Katalysatorsystemen mit Propylenoxid (A-1), PC (BH), einer Reaktionstemperatur von T=75°C, einer Rektionszeit von t=5 h, einem molaren Verhältnis (A-1)/n(BH) = 8.**

| # | Katalysator | organisch | n | D | X (A-1) [%] | Mₙ [g/mol] | cPC^{a)} [Gew.-%] |
|---|---|---|---|---|---|---|---|
| 11 | **C-5** | Ja | 2 | 1,3 | 100 | 1381 | 2,7 |
| 15 Vgl. | CF₃SO₃H | Ja | 1 | 1,0 | 100 | 808 | 39,0 |

| | | | | | | | |
|---|---|---|---|---|---|---|---|
| a) Neubildung cyclisches Propylencarbonat cPC, bezogen auf eingesetztes Polycarbonatpolyols PC, abzüglich 3 Gew.-% cPC aus Startermaterial. | | | | | | | |

**Tabelle 9: Test auf Aktivität des erfindungsgemäßen Katalysatorsystems C-7 mit Styroloxid (A-1), PEG (BH), einer Reaktionstemperatur von T=70°C, einem molaren Verhältnis (A-1)/(BH) =4.**

| # | Katalysator | organisch | n | D | X (A-1) [%] | Mₙ [g/mol] |
|---|---|---|---|---|---|---|
| 13 | **C-7** | ja | 2 | 1,0 | 28 | 1135 |

**Tabelle 10: Copolymerisation von Propylenoxid (A-1) mit Comonomeren (A-2, A-4, A-6, oder A-7) in Gegenwart von Katalysator (C-5) bei Temperaturen von 80-100°C.**

| # | Comonomere | Katalysator | organisch | n | D | T [°C] | M [g/mol] |
|---|---|---|---|---|---|---|---|
| 26 | **A-1,A-6** | **C-5** | ja | 2 | 1,3 | 80-100 | 1107 (M_{P}) |
| 27 | **A-1,A-2** | **C-5** | ja | 2 | 1,3 | 80 | 1349 (M_{P}) |
| 28 | **A-1,A-2** | **C-5** | ja | 2 | 1,3 | 80 | 1295 (M_{P}) |
| 29 | **A-1,A-7** | **C-5** | ja | 2 | 1,3 | 100 | 1132 (M_{P}) |
| 30 | **A-1,A-4** | **C-5** | ja | 2 | 1,3 | 60-70 | 1652 (Mₙ) |

**Tabelle 11: Test auf Aktivität und Selektivität von erfindungsgemäßen und vergleichenden Katalysatorsystemen mit Alkylenoxid (A-1), H-funktionellen Starterverbindungen (BH) mit einem molaren Verhältnis (A-1)/n(BH) = 8.**

| # | (A-1) | (BH) | Katalysator | Lösungsmittel | X(A-1) [%] | Mₙ [g/mol] | PDI | Nebenkomponenten [Gew.-%] |
|---|---|---|---|---|---|---|---|---|
| 11 | PO | PC | **C-5** | - | 100 | 1381 | 1,3 | 2,7^{a)} / -^{b)} |
| 15 Vgl. | PO | PC | CF3SO3H | - | 100 | 808 | 1,6 | 39,0^{a)} / -^{b)} |
| 24 | PO | PC | **C-5** | THF | 100 | 1633 | 1,3 | 1,2^{a)} / 8,2^{b}) |
| 25 Vgl. | PO | PC | CF3SO3H | THF | 100 | 1340 | 1,4 | 22,3^{a)} / 13,1^{b)} |
| 8 | EO | PET | **C-5** | - | 100 | 740 | 1,2 | 0^{a)} / 0,6^{b)} |
| 9 | EO | pFA | **C-5** | cPC | 100 | 1409 | 1,9 | 0^{a)} / 8,6^{b)} |

| | | | | | | | | |
|---|---|---|---|---|---|---|---|---|
| a) Bildung cyclisches Propylencarbonat (cPC), bezogen auf eingesetztes Polycarbonatpolyol, abzüglich 3 Gew.-% cPC aus Startermaterial. b) Der Anteil an Nebenkomponenten ohne cPC bezogen auf das Reaktionsgemisch in Gew.-%. | | | | | | | | |

## Patentansprüche

1. Verfahren zur Anlagerung einer Verbindung (A) an eine H-funktionelle Starterverbindung (BH) in Gegenwart eines Katalysators
, wobei die mindestens eine Verbindung (A) ausgewählt wird aus mindestens einer Gruppe bestehend aus Alkylenoxid (A-1), Lacton (A-2), Lactid (A-3), cyclisches Acetal (A-4), Lactam (A-5), cyclisches Anhydrid (A-6) und sauerstoffhaltige Heterocyclenverbindung (A-7) verschieden von (A-1), (A-2), (A-3), (A-4) und (A-6),
**dadurch gekennzeichnet, dass**
der Katalysator eine organische, n-protonige Bronsted-Säure (C) umfasst, wobei n ≥ 2 und Element der natürlichen Zahl und der Protolysegrad D 0<D<n ist mit n als maximale Zahl an übertragbaren Protonen und D als berechneter Protonenanteil der organischen, n-protonigen Bronsted-Säure (C), wobei die organische, n-protonige Bronsted-Säure (C) eine berechnete Molmasse von ≤ 1200 g/mol aufweist.

2. Verfahren gemäß Anspruch 1, wobei die Verbindung (A) ausgewählt wird aus mindestens einer Gruppe bestehend aus Alkylenoxid (A-1), Lacton (A-2), cyclisches Acetal (A-4) und cyclisches Anhydrid (A-6).

3. Verfahren gemäß Anspruch 1 oder 2, wobei die organische, n-protonige Bronsted-Säure (C) eine Sulfonsäure ist.

4. Verfahren gemäß einem der Ansprüche 1 bis 3, wobei die maximale Zahl an übertragbaren Protonen n n = 2, 3 oder 4 beträgt.

5. Verfahren gemäß Anspruch 4, wobei der Protolysegrad D für zweiprotonige Säuren mit n = 2 0,2 bis 1,9, für dreiprotonige Säuren mit n = 3 0,3 bis 2,8 und für vierprotonige Säuren mit n = 4 0,4 bis 3,7 beträgt.

6. Verfahren gemäß einem der Ansprüche 1 bis 5, wobei die organische, n-protonige Bronsted-Säure (C) mit dem Protolysegrad 0<D<n durch Säure-Base-Reaktionen mit Protonenübergang durch
(α) Zugabe geeigneter Mengen von geeigneten Bronsted-Basen (E) zu den organischen, n-protonigen Bronsted-Säuren oder
(β) Zugabe geeigneter Mengen von geeigneten Bronsted-Säuren (E'H) zu den Salzen der organischen n-protonigen Bronsted-Säuren erhalten wird.

7. Verfahren gemäß Anspruch 6, wobei die organische, n-protonige Bronsted-Säure (C) mit dem Protolysegrad 0<D<n durch Säure-Base-Reaktionen mit Protonenübergang in Schritt
(α) durch Zugabe Bronsted-Basen (E) mit einem pK_{b}(E)-Wert von ≤10, bevorzugt mit einem pK_{b}(E)-Wert von ≤8 und ganz besonders bevorzugt mit einem pK_{b}(E)-Wert von ≤5 zu der vollständig protonierten Sulfonsäuren oder
(β) durch Zugabe starker Bronsted-Säuren (E'H) mit einem pKₛ(E'H)-Wert von ≤1 zu dem Metallsalzes einer Sulfonsäure erhalten wird.

8. Verfahren gemäß einem der Ansprüche 1 bis 7, wobei die mindestens eine Verbindung (A) ausgewählt wird aus der Gruppe bestehend aus Ethylenoxid, Propylenoxid, Styroloxid , Allylglycidylether, ε-Caprolacton, Propiolacton, β-Butyrolacton, γ-Butyrolacton, ε-Caprolactam, 1,3-Dioxolan, 1,4-Dioxan, Tetrahydrofuran und 1,3,5-Trioxan.

9. Verfahren gemäß einem der Ansprüche 1 bis 8, wobei die Verbindung (BH) eine oder mehrere Verbindungen ist und ausgewählt wird aus der Gruppe bestehend aus ein- oder mehrwertige Alkohole, mehrwertige Amine, mehrwertige Thiole, Aminoalkohole, Thioalkohole, Hydroxyester, Polyetherpolyole, Polyesterpolyole, Polyesteretherpolyole, Polyethercarbonatpolyole, Polycarbonatpolyole, Polycarbonate, Polyacetalen, polymere Formaldehydverbindungen, Polyethylenimine, Polyetheramine, Polytetrahydrofurane, Polytetrahydrofuranamine, Polyetherthiole, Polyacrylatpolyole, Ricinusöl, das Mono- oder Diglycerid von Ricinolsäure, Monoglyceride von Fettsäuren, chemisch modifizierte Mono-, Di- und/oder Triglyceride von Fettsäuren und C1-C24 Alkyl-Fettsäureester, die im Mittel mindestens 2 OH-Gruppen pro Molekül enthalten.

10. n-protonige Bronsted-Säure (C) mit einem Protolysegrad D 0<D<n, wobei n die maximale Zahl an übertragbaren Protonen mit n = 2, 3 oder 4 und D der berechnete Protonenanteil der organischen, n-protonigen Bronsted-Säure (C) ist,
**dadurch gekennzeichnet, dass**
der Protolysegrad D für zweiprotonige Säuren mit n = 2 0,2 bis 1,9, für dreiprotonige Säuren mit n = 3 0,3 bis 2,8 und für vierprotonige Säuren mit n = 4 0,4 bis 3,7 beträgt,
wobei die organische, n-protonige Bronsted-Säure (C) mit dem Protolysegrad 0<D<n durch Säure-Base-Reaktionen mit Protonenübergang durch
(α) Zugabe geeigneter Mengen von mindestens einer geeigneten Bronsted-Base (E) zu der mindestens einen organischen, n-protonigen Bronsted-Säure erhalten wird, wobei die Bronsted-Base (E) mindestens ein Kation (F) enthält, das ausgewählt ist aus der Gruppe bestehend aus Alkalimetall-, Erdalkalimetall-, Halbmetall-haltige-, Übergangsmetall-, Lanthanoidmetall-, aliphatische Ammonium - haltige und Phosphonium - haltige und Sulfonium - haltige - Kationen
oder
Zugabe geeigneter Mengen von mindestens einer geeigneten Bronsted-Säure (E'H) zu dem Salz der mindestens einen organischen n-protonigen Bronsted-Säure erhalten wird, wobei die Salze der organischen n-protonigen Bronsted-Säure mindestens ein Kation (F') enthält, das ausgewählt ist aus der Gruppe bestehend aus Alkalimetall-, Erdalkalimetall-, Halbmetall-haltige, Übergangsmetall-haltige, Lanthanoidmetall-haltige, aliphatische Ammonium - haltige und Phosphonium - haltige und Sulfonium - haltige - Kationen, wobei die n-protonige Bronsted-Säure (C) mindestens eine Sulfonsäure ist und wobei die mindestens eine Sulfonsäure ausgewählt wird aus der Gruppe bestehend aus 1,5-Naphthalindisulfonsäure, 2,6-Naphthalindisulfonsäure und 1,3-Benzoldisulfonsäure, bevorzugt 1,5-Naphthalindisulfonsäure, 2,6-Naphthalindisulfonsäure und ganz besonders bevorzugt 2,6-Naphthalindisulfonsäure.

11. n-protonige Bronsted-Säure (C) gemäß Anspruch 10, wobei das Kation (F) ausgewählt wird aus der Gruppe bestehend aus Lithiumkation, Natriumkation, Kaliumkation, Rubidiumkation, Cäsiumkation, Magnesiumkation, Calciumkation, Strontiumkation, Bariumkation, Scandiumkation, Titankation, Zinkkation, Aluminiumkation, aliphatischen primären Ammonium-Ionen, aliphatischen sekundären Ammonium-Ionen, aliphatischen tertiären Ammonium-Ionen, aliphatischen quaternären Ammonium-Ionen, Phosphonium Ionen, Sulfonium-Ionen und Sulfoxonium Ionen, bevorzugt aus Lithiumkation, Natriumkation, Kaliumkation, Magnesiumkation, Calciumkation, quaternären Ammonium-Ionen, und Triphenylphosphonium Ionen und besonders bevorzugt aus Natriumkation, Kaliumkation, Magnesiumkation und n-Butyl-Ammoniumion.

12. n-protonige Bronsted-Säure (C) gemäß einem der Ansprüche 10 bis 11, wobei das Kation (F') ausgewählt wird aus der Gruppe bestehend aus Lithiumkation, Natriumkation, Kaliumkation, Rubidiumkation, Cäsiumkation, Magnesiumkation, Calciumkation, Strontiumkation, Bariumkation, Scandiumkation, Titankation, Zinkkation, Aluminiumkation, aliphatischen primären Ammonium-Ionen, aliphatischen sekundären Ammonium-Ionen, aliphatischen tertiären Ammonium-Ionen, aliphatischen quaternären Ammonium-Ionen, Phosphonium Ionen, Sulfonium-Ionen und Sulfoxonium Ionen, bevorzugt aus Lithiumkation, Natriumkation, Kaliumkation, Magnesiumkation, Calciumkation, quaternären Ammonium-Ionen, und Triphenylphosphonium Ionen und besonders bevorzugt aus Natriumkation, Kaliumkation, Magnesiumkation und n-Butyl-Ammoniumion.

13. n-protonige Bronsted-Säure (C) gemäß einem der Ansprüche 10 bis 12, wobei die mindestens eine Bronsted-Base (E) ausgewählt ist aus der Gruppe bestehend aus Lithiumhydroxid, Natriumhydroxid, Kaliumhydroxid, Rubidiumhydroxid, Cäsiumhydroxid, Magnesiumhydroxid, Calciumhydroxid, Strontiumhydroxid, Bariumhydroxid, Scandiumhydroxid, Titanhydroxid, Zinkhydroxid, Aluminiumhydroxid, aliphatischen primären Ammoniumhydroxiden, aliphatischen sekundären Ammoniumhydroxiden, aliphatischen tertiären Ammoniumhydroxiden, aliphatischen quaternären Ammoniumhydroxiden, Phosphoniumhydroxiden, aliphatischen primären Ammoniumalkoholaten, aliphatischen sekundären Ammoniumalkoholaten, aliphatischen tertiären Ammoniumalkoholaten, aliphatischen quaternären Ammoniumalkoholaten, Phosphoniumalkoholaten, Butyllithium, Kalium-tert-butanolat, 1,8-Diazabicyclo[5.4.0]undec-7-en (DBU), 1,5-Diazabicyclo(4.3.0)non-5-en (DBN), primären aliphatischen Aminen, sekundären aliphatischen Aminen, tertiären aliphatischen Aminen, primären cycloaliphatischen Aminen, sekundären cycloaliphatischen Aminen, tertiären cycloaliphatischen Aminen und Phosphoniumalkoholaten bevorzugt aus Natriumhydroxid, Kaliumhydroxid, Lithiumhydroxid, Magnesiumhydroxid Calciumhydroxid, aliphatischen quaternären Ammoniumalkoholaten, Phosphoniumalkoholaten, Ammoniak, Triethylamin, Trimethylamin, Diethylamin, Propylamin, Methylamin, Dimethylamin, Ethylamin, Ethylenediamin, 1,3-Diaminpropane, Putrescin, 1,5-Diaminopentan, Hexamethylendiamin, 1,2-Diaminopropane, Diaminocyclohexan, n-Propylamin, Di-n-Propylamin, Tri-n-Propylamin, Isopropylamin, Diisopropylamin, n-Butylamin, Di-n-butylamin, Tri-n-butylamin, Di-isobutylamin, 2-Aminobutan, 2-Ethylhexylamin, Di-2-Ethylhexylamin, Cyclohexylamin, Dicyclohexylamin, Dimethylaminopropylamin Triethylendiamin, 1,8-Diazabicyclo[5.4.0]undec-7-en (DBU) und 1,5-Diazabicyclo(4.3.0)non-5-en (DBN) besonders bevorzugt aus Natriumhydroxid, Kaliumhydroxid, Lithiumhydroxid, Magnesiumhydroxid, Tetra-(n-Butyl)ammoniummethanolat, Tetra-(n-Butyl)ammoniumethanolat und Tetra-(n-Butyl)ammoniumisopropylat.

14. n-protonige Bronsted-Säure (C) gemäß einem der Ansprüche 10 bis 13, wobei die mindestens eine Bronsted-Säure (E'H) ausgewählt ist aus der Gruppe bestehend aus aliphatischen fluorierten Sulfonsäuren, aromatischen fluorierten Sulfonsäuren, Trifluoromethansulfonsäure, Perchlorsäure, Chlorwasserstoffsäure, Bromwasserstoffsäure, Jodwasserstoffsäure, Fluorsulfonsäure, Bis(trifluoromethan)sulfonimid, Hexafluorantimonsäure, Pentacyanocyclopentadien, Pikrinsäure, Schwefelsäure, Salpetersäure, Trifluoressigsäure, Methansulfonsäure, Paratoluolsulfonsäure, aromatische Sulfonsäuren und aliphatische Sulfonsäuren, bevorzugt aus Trifluoromethansulfonsäure, Perchlorsäure, Chlorwasserstoffsäure, Bromwasserstoffsäure, Jodwasserstoffsäure, Fluorsulfonsäure, Bis(trifluoromethan)sulfonimid, Hexafluorantimonsäure, Pentacyanocyclopentadien, Pikrinsäure, Schwefelsäure, Salpetersäure, Trifluoressigsäure, Methansulfonsäure, Paratoluolsulfonsäure, Methansulfonsäure und Paratoluolsulfonsäure besonders bevorzugt aus Trifluoromethansulfonsäure, Perchlorsäure, Chlorwasserstoffsäure, Bromwasserstoffsäure, Jodwasserstoffsäure, Bis(trifluoromethan)-sulfonimid, Pentacyanocyclopentadien, Schwefelsäure, Salpetersäure und Trifluoressigsäure.

15. Sulfonsäure gemäß einem der Ansprüche 10 bis 14, wobei der Protolysegrad D 0,8 bis 1,8, bevorzugt 1,1 bis 1,7, beträgt.

## Claims

1. Process for addition of a compound (A) onto an H-functional starter compound (BH) in the presence of a catalyst,
wherein the at least one compound (A) is selected from at least one group consisting of alkylene oxide (A-1), lactone (A-2), lactide (A-3), cyclic acetal (A-4), lactam (A-5), cyclic anhydride (A-6) and oxygen-containing heterocycle compound (A-7) distinct from (A-1), (A-2), (A-3), (A-4) and (A-6),
**characterized in that**
the catalyst comprises an organic, n-protic Bronsted acid (C), wherein n ≥ 2 and is an element of the natural numbers and the degree of protolysis D is 0<D<n where n is the maximum number of transferrable protons and D is the calculated proton fraction of the organic, n-protic Bronsted acid (C),
wherein the organic, n-protic Bronsted acid (C) has a calculated molar mass of ≤ 1200 g/mol.

2. Process according to Claim 1, wherein the compound (A) is selected from at least one group consisting of alkylene oxide (A-1), lactone (A-2), cyclic acetal (A-4) and cyclic anhydride (A-6).

3. Process according to Claim 1 or 2, wherein the organic, n-protic Bronsted acid (C) is a sulfonic acid.

4. Process according to any of Claims 1 to 3, wherein the maximum number of transferable protons n is n = 2, 3 or 4.

5. Process according to Claim 4, wherein the degree of protolysis D for diprotic acids where n = 2 is 0.2 to 1.9, for triprotic acids where n = 3 is 0.3 to 2.8 and for tetraprotic acids where n = 4 is 0.4 to 3.7.

6. Process according to any of Claims 1 to 5, wherein the organic, n-protic Bronsted acid (C) having the degree of protolysis 0<D<n is obtained by acid-base reactions with proton transfer by
(α) addition of suitable amounts of suitable Bronsted bases (E) to the organic, n-protic Bronsted acids or
(β) addition of suitable amounts of suitable Bronsted acids (E'H) to the salts of the organic, n-protic Bronsted acids.

7. Process according to Claim 6, wherein the organic, n-protic Bronsted acid (C) having the degree of protolysis 0<D<n is obtained by acid-base reactions with proton transfer in step
(α) by addition of Bronsted bases (E) having a pK_{b}(E) of ≤ 10, preferably having a pK_{b}(E) of ≤ 8 and very particularly preferably having a pK_{b}(E) of ≤ 5 to the completely protonated sulfonic acids or
(β) by addition of strong Bronsted acids (E'H) having a pKₛ(E'H) of ≤ 1 to the metal salt of a sulfonic acid.

8. Process according to any of Claims 1 to 7, wherein the at least one compound (A) is selected from the group consisting of ethylene oxide, propylene oxide, styrene oxide, allyl glycidyl ether, ε-caprolactone, propiolactone, β-butyrolactone, γ-butyrolactone, ε-caprolactam, 1,3-dioxolane, 1,4-dioxane, tetrahydrofuran and 1,3,5-trioxane.

9. Process according to any of Claims 1 to 8, wherein the compound (BH) is one or more compounds and is selected from the group consisting of mono- or polyvalent alcohols, polyvalent amines, polyvalent thiols, amino alcohols, thio alcohols, hydroxy esters, polyether polyols, polyester polyols, polyester ether polyols, polyether carbonate polyols, polycarbonate polyols, polycarbonates, polyacetals, polymeric formaldehyde compounds, polyethyleneimines, polyetheramines, polytetrahydrofurans, polytetrahydrofuranamines, polyether thiols, polyacrylate polyols, castor oil, the mono- or diglyceride of ricinoleic acid, monoglycerides of fatty acids, chemically modified mono-, di- and/or triglycerides of fatty acids and C1-C24 alkyl fatty acid esters containing on average at least 2 OH groups per molecule.

10. n-protic Bronsted acid (C) having a degree of protolysis D of 0<D<n, wherein n is the maximum number of transferable protons where n = 2, 3 or 4 and D is the calculated proton fraction of the organic, n-protic Bronsted acid (C),
**characterized in that**
the degree of protolysis D for diprotic acids where n = 2 is 0.2 to 1.9, for triprotic acids where n = 3 is 0.3 to 2.8 and for tetraprotic acids where n = 4 is 0.4 to 3.7,
wherein the organic, n-protic Bronsted acid (C) having the degree of protolysis 0<D<n is obtained by acid-base reactions with proton transfer by
(α) addition of suitable amounts of at least one suitable Bronsted base (E) to the at least one organic, n-protic Bronsted acid, wherein the Bronsted base (E) contains at least one cation (F) selected from the group consisting of alkali metal-containing, alkaline earth metal-containing, metalloid-containing, transition metal-containing, lanthanoid metal-containing, aliphatic ammonium-containing and phosphonium-containing and sulfonium-containing cations or
addition of suitable amounts of at least one suitable Bronsted base (E'H) to the salt of the at least one organic, n-protic Bronsted acid, wherein the salts of the organic, n-protic Bronsted acid contains at least one cation (F') selected from the group consisting of alkali metal-containing, alkaline earth metal-containing, metalloid-containing, transition metal-containing, lanthanoid metal-containing, aliphatic ammonium-containing and phosphonium-containing and sulfonium-containing cations, wherein the n-protic Bronsted acid (C) is at least one sulfonic acid and wherein the at least one sulfonic acid is selected from the group consisting of 1,5-naphthalenedisulfonic acid, 2,6-naphthalenedisulfonic acid and 1,3-benzenedisulfonic acid, preferably 1,5-naphthalenedisulfonic acid, 2,6-naphthalenedisulfonic acid and very particularly preferably 2,6-naphthalenedisulfonic acid.

11. n-protic Bronsted acid (C) according to Claim 10, wherein the cation (F) is selected from the group consisting of lithium cation, sodium cation, potassium cation, rubidium cation, cesium cation, magnesium cation, calcium cation, strontium cation, barium cation, scandium cation, titanium cation, zinc cation, aluminium cation, aliphatic primary ammonium ions, aliphatic secondary ammonium ions, aliphatic tertiary ammonium ions, aliphatic quaternary ammonium ions, phosphonium ions, sulfonium ions and sulfoxonium ions, preferably from lithium cation, sodium cation, potassium cation, magnesium cation, calcium cation, quaternary ammonium ions and triphenylphosphonium ions and particularly preferably from sodium cation, potassium cation, magnesium cation and n-butylammonium ion.

12. n-protic Bronsted acid (C) according to either of Claims 10 to 11, wherein the cation (F') is selected from the group consisting of lithium cation, sodium cation, potassium cation, rubidium cation, cesium cation, magnesium cation, calcium cation, strontium cation, barium cation, scandium cation, titanium cation, zinc cation, aluminium cation, aliphatic primary ammonium ions, aliphatic secondary ammonium ions, aliphatic tertiary ammonium ions, aliphatic quaternary ammonium ions, phosphonium ions, sulfonium ions and sulfoxonium ions, preferably from lithium cation, sodium cation, potassium cation, magnesium cation, calcium cation, quaternary ammonium ions and triphenylphosphonium ions and particularly preferably from sodium cation, potassium cation, magnesium cation and n-butylammonium ion.

13. n-protic Bronsted acid (C) according to any of Claims 10 to 12, wherein the at least one Bronsted base (E) is selected from the group consisting of lithium hydroxide, sodium hydroxide, potassium hydroxide, rubidium hydroxide, cesium hydroxide, magnesium hydroxide, calcium hydroxide, strontium hydroxide, barium hydroxide, scandium hydroxide, titanium hydroxide, zinc hydroxide, aluminium hydroxide, aliphatic primary ammonium hydroxides, aliphatic secondary ammonium hydroxides, aliphatic tertiary ammonium hydroxides, aliphatic quaternary ammonium hydroxides, phosphonium hydroxides, aliphatic primary ammonium alkoxides, aliphatic secondary ammonium alkoxides, aliphatic tertiary ammonium alkoxides, aliphatic quaternary ammonium alkoxides, phosphonium alkoxides, butyllithium, potassium tert-butoxide, 1,8-diazabicyclo[5.4.0]undec-7-ene (DBU), 1,5-diazabicyclo(4.3.0)non-5-ene (DBN), primary aliphatic amines, secondary aliphatic amines, tertiary aliphatic amines, primary cycloaliphatic amines, secondary cycloaliphatic amines, tertiary cycloaliphatic amines and phosphonium alkoxides, preferably from sodium hydroxide, potassium hydroxide, lithium hydroxide, magnesium hydroxide, calcium hydroxide, aliphatic quaternary ammonium alkoxides, phosphonium alkoxides, ammonia, triethylamine, trimethylamine, diethylamine, propylamine, methylamine, dimethylamine, ethylamine, ethylenediamine, 1,3-diaminopropanes, putrescine, 1,5-diaminopentane, hexamethylenediamine, 1,2-diaminopropanes, diaminocyclohexane, n-propylamine, din-propylamine, tri-n-propylamin, isopropylamine, diisopropylamine, n-butylamine, di-n-butylamine, tri-n-butylamine, diisobutylamine, 2-aminobutane, 2-ethylhexylamine, di-2-ethylhexylamine, cyclohexylamine, dicyclohexylamine, dimethylaminopropylamine, triethylenediamine, 1,8-diazabicyclo[5.4.0]undec-7-ene (DBU) and 1,5-diazabicyclo(4.3.0)non-5-ene (DBN), particularly preferably from sodium hydroxide, potassium hydroxide, lithium hydroxide, magnesium hydroxide, tetra(n-butyl)ammonium methoxide, tetra(n-butyl)ammonium ethoxide and tetra(n-butyl)ammonium isopropoxide.

14. n-protic Bronsted acid (C) according to any of Claims 10 to 13, wherein the at least one Bronsted acid (E'H) is selected from the group consisting of aliphatic fluorinated sulfonic acids, aromatic fluorinated sulfonic acids, trifluoromethanesulfonic acid, perchloric acid, hydrochloric acid, hydrobromic acid, hydroiodic acid, fluorosulfonic acid, bis(trifluoromethane)sulfonimide, hexafluorantimonic acid, pentacyanocyclopentadiene, picric acid, sulfuric acid, nitric acid, trifluoroacetic acid, methanesulfonic acid, paratoluenesulfonic acid, aromatic sulfonic acids and aliphatic sulfonic acids, preferably from trifluoromethanesulfonic acid, perchloric acid, hydrochloric acid, hydrobromic acid, hydroiodic acid, fluorosulfonic acid, bis(trifluoromethane)sulfonimide, hexafluorantimonic acid, pentacyanocyclopentadiene, picric acid, sulfuric acid, nitric acid, trifluoroacetic acid, methanesulfonic acid, paratoluenesulfonic acid, methanesulfonic acid and paratoluenesulfonic acid, particularly preferably from trifluoromethanesulfonic acid, perchloric acid, hydrochloric acid, hydrobromic acid, hydroiodic acid, bis(trifluoromethane)sulfonimide, pentacyanocyclopentadiene, sulfuric acid, nitric acid and trifluoroacetic acid.

15. Sulfonic acid according to any of Claims 10 to 14, wherein the degree of protolysis D is 0.8 to 1.8, preferably 1.1 to 1.7.

## Revendications

1. Procédé pour l'addition d'un composé (A) sur un composé de départ (BH) fonctionnalisé par H en présence d'un catalyseur, l'au moins un composé (A) étant choisi dans au moins un groupe constitué par un oxyde d'alkylène (A-1), une lactone (A-2), un lactide (A-3), un acétal cyclique (A-4), un lactame (A-5), un anhydride cyclique (A-6) et un composé hétérocyclique contenant de l'oxygène (A-7) différent de (A-1), (A-2), (A-3), (A-4), et (A-6),
**caractérisé en ce que** le catalyseur comprend un acide de Bronsted (C) n-protoné, organique, avec n ≥ 2 et n étant élément de nombre entier et le degré de protolyse D étant 0 < D < n avec n en tant que nombre maximum de protons transférables et D en tant que proportion de protons calculée de l'acide de Bronsted (C) n-protoné, organique, l'acide de Bronsted (C) n-protoné, organique présentant une masse molaire calculée ≤ 1200 g/mole.

2. Procédé selon la revendication 1, le composé (A) étant choisi dans au moins un groupe constitué par un oxyde d'alkylène (A-1), une lactone (A-2), un acétal cyclique (A-4) et un anhydride cyclique (A-6).

3. Procédé selon la revendication 1 ou 2, l'acide de Bronsted (C) n-protoné, organique étant un acide sulfonique.

4. Procédé selon l'une quelconque des revendications 1 à 3, le nombre maximum de protons transférables n étant n = 2, 3 ou 4.

5. Procédé selon la revendication 4, le degré de protolyse D pour des acides à deux protons avec n = 2 étant de 0,2 à 1,9, pour des acides à trois protons avec n = 3 étant de 0,3 à 2,8 et pour des acides à quatre protons avec n = 4 étant de 0,4 à 3,7.

6. Procédé selon l'une quelconque des revendications 1 à 5, l'acide de Bronsted (C) n-protoné, organique avec le degré de protolyse 0 < D < n étant obtenu par réactions acide-base avec transfert de protons par
(α) ajout de quantités appropriées de bases de Bronsted (E) appropriées aux acides de Bronsted n-protonés, organiques ou
(β) ajout de quantités appropriées d'acides de Bronsted (E'H) appropriés aux sels des acides de Bronsted n-protonés, organiques.

7. Procédé selon la revendication 6, l'acide de Bronsted (C) n-protoné, organique avec le degré de protolyse 0 < D < n étant obtenu par réactions acide-base avec transfert de protons dans l'étape
(α) par ajout de bases de Bronsted (E) dotées d'une valeur de pK_{b}(E) ≤ 10, préférablement dotées d'une valeur de pK_{b}(E) ≤ 8 et tout particulièrement préférablement dotées d'une valeur de pK_{b}(E) ≤ 5 aux acides sulfoniques totalement protonés ou
(β) par ajout d'acides de Bronsted (E'H) forts dotés d'une valeur de pKₛ(E'H) ≤ 1 au sel métallique d'un acide sulfonique.

8. Procédé selon l'une quelconque des revendications 1 à 7, l'au moins un composé (A) étant choisi dans le groupe constitué par l'oxyde d'éthylène, l'oxyde de propylène, l'oxyde de styrène, l'allylglycidyléther, la ε-caprolactone, la propriolactone, la β-butyrolactone, la γ-butyrolactone, le ε-caprolactame, le 1,3-dioxolanne, le 1,4-dioxanne, le tétrahydrofuranne et le 1,3,5-trioxanne.

9. Procédé selon l'une quelconque des revendications 1 à 8, le composé (BH) étant un ou plusieurs composés et étant choisi dans le groupe constitué par les alcools monovalents et polyvalents, les amines polyvalentes, les thiols polyvalents, les aminoalcools, les thioalcools, les hydroxyesters, les polyéther polyols, les polyesters polyols, les polyesteréther polyols, les polyéthercarbonate polyols, les polycarbonate polyols, les polycarbonates, les polyacétals, les composés polymériques du formaldéhyde, les polyéthylènimines, les polyétheramines, les polytétrahydrofurannes, les polytétrahydrofurannamines, les polyétherthiols, les polyacrylate polyols, l'huile de ricin, le monoglycéride ou le diglycéride de l'acide ricinoléïque, les monoglycérides d'acides gras, les monoglycérides, les diglycérides et/ou les triglycérides chimiquement modifiés d'acides gras et les esters d'acides gras de C₁₋₂₄-alkyle, qui contiennent en moyenne au moins 2 groupes OH par molécule.

10. Acide de Bronsted (C) n-protoné avec un degré de protolyse D 0 < D < n, n étant le nombre maximum de protons transférables avec n = 2, 3 ou 4 et D étant la proportion de protons calculée de l'acide de Bronsted (C) n-protoné, organique,
**caractérisé en ce que** le degré de protolyse D pour des acides à deux protons avec n = 2 est de 0,2 à 1,9, pour des acides à trois protons avec n = 3 est de 0,3 à 2,8 et pour des acides à quatre protons avec n = 4 étant de 0,4 à 3,7,
l'acide de Bronsted (C) n-protoné, organique avec le degré de protolyse 0 < D < n étant obtenu par des réactions acide-base avec transfert de protons par
(α) ajout de quantités appropriées d'au moins une base de Bronsted (E) appropriée à l'au moins un acide de Bronsted n-protoné, organique, la base de Bronsted (E) contenant au moins un cation (F), qui est choisi dans le groupe constitué par les cations de métal alcalin, les cations de métal alcalino-terreux, les cations contenant un semi-métal, les cations de métal de transition, les cations de métal lanthanoïde, les cations contenant un ammonium et contenant un phosphonium et contenant un sulfonium aliphatiques
ou
ajout de quantités appropriées d'au moins un acide de Bronsted (E'H) approprié au sel de l'au moins un acide Bronsted n-protoné, organique, les sels de l'acide de Bronsted n-protoné, organique contenant au moins un cation (F'), qui est choisi dans le groupe constitué par les cations de métal alcalin, les cations de métal alcalino-terreux, les cations contenant un semi-métal, les cations contenant un métal de transition, les cations contenant un métal lanthanoïde, les cations contenant un ammonium et contenant un phosphonium et contenant un sulfonium aliphatiques, l'acide de Bronsted (C) n-protoné étant au moins un acide sulfonique et l'au moins un acide sulfonique étant choisi dans le groupe constitué par l'acide 1,5-naphtalènedisulfonique, l'acide 2,6-naphtalènedisulfonique, l'acide 1,3-benzènedisulfonique, préférablement l'acide 1,5-naphtalènedisulfonique, l'acide 2,6-naphtalènedisulfonique et tout particulièrement préférablement l'acide 2,6-naphtalènedisulfonique.

11. Acide de Bronsted (C) n-protoné selon la revendication 10, le cation (F) étant choisi dans le groupe constitué par le cation lithium, le cation sodium, le cation potassium, le cation rubidium, le cation césium, le cation magnésium, le cation calcium, le cation strontium, le cation baryum, le cation scandium, le cation titane, le cation zinc, le cation aluminium, les ions ammonium aliphatiques primaires, les ions ammonium aliphatiques secondaires, les ions ammonium aliphatiques tertiaires, les ions ammonium aliphatiques quaternaires, les ions phosphonium, les ions sulfonium et les ions sulfoxonium, préférablement constitué par le cation lithium, le cation sodium, le cation potassium, le cation magnésium, le cation calcium, les ions ammonium quaternaires, et les ions triphénylphosphonium et particulièrement préférablement constitué par le cation sodium, le cation potassium, le cation magnésium et l'ion n-butylammonium.

12. Acide de Bronsted (C) n-protoné selon l'une quelconque des revendications 10 et 11, le cation (F') étant choisi dans le groupe constitué par le cation lithium, le cation sodium, le cation potassium, le cation rubidium, le cation césium, le cation magnésium, le cation calcium, le cation strontium, le cation baryum, le cation scandium, le cation titane, le cation zinc, le cation aluminium, les ions ammonium aliphatiques primaires, les ions ammonium aliphatiques secondaires, les ions ammonium aliphatiques tertiaires, les ions ammonium aliphatiques quaternaires, les ions phosphonium, les ions sulfonium et les ions sulfoxonium, préférablement constitué par le cation lithium, le cation sodium, le cation potassium, le cation magnésium, le cation calcium, les ions ammonium quaternaires, et les ions triphénylphosphonium et particulièrement préférablement constitué par le cation sodium, le cation potassium, le cation magnésium et l'ion n-butylammonium.

13. Acide de Bronsted (C) n-protoné selon l'une quelconque des revendications 10 à 12, l'au moins une base de Bronsted (E) étant choisie dans le groupe constitué par l'hydroxyde de lithium, l'hydroxyde de sodium, l'hydroxyde de potassium, l'hydroxyde de rubidium, l'hydroxyde césium, l'hydroxyde de magnésium, l'hydroxyde de calcium, l'hydroxyde strontium, l'hydroxyde de baryum, l'hydroxyde de scandium, l'hydroxyde de titane, l'hydroxyde de zinc, l'hydroxyde d'aluminium, les hydroxydes d'ammonium aliphatiques primaires, les hydroxydes d'ammonium aliphatiques secondaires, les hydroxydes d'ammonium aliphatiques tertiaires, les hydroxydes d'ammonium aliphatiques quaternaires, les hydroxydes de phosphonium, les alcoolates d'ammonium aliphatiques primaires, les alcoolates d'ammonium aliphatiques secondaires, les alcoolates d'ammonium aliphatiques tertiaires, les alcoolates d'ammonium aliphatiques quaternaires, les alcoolates de phosphonium, le butyllithium, le tert-butanolate de potassium, le 1,8-diazabicyclo[5.4.0]undec-7-ène (DBU), le 1,5-diazabicyclo[4.3.0]non-5-ène (DBN), les amines aliphatiques primaires, les amines aliphatiques secondaires, les amines aliphatiques tertiaires, les amines cycloaliphatiques primaires, les amines cycloaliphatiques secondaires, les amines cycloaliphatiques tertiaires et les alcoolates de phosphonium, préférablement constitué par l'hydroxyde de sodium, l'hydroxyde de potassium, l'hydroxyde de lithium, l'hydroxyde de magnésium, l'hydroxyde de calcium, les alcoolates d'ammonium aliphatiques quaternaires, les alcoolates de phosphonium, l'ammoniac, la triéthylamine, la triméthylamine, la diéthylamine, la propylamine, la méthylamine, la diméthylamine, l'éthylamine, l'éthylènediamine, le 1,3-diaminopropane, la putrescine, le 1,5-diaminopentane, l'hexamethylènediamine, le 1,2-diaminopropane, le diaminocyclohexane, la n-propylamine, la di-n-propylamine, la tri-n-propylamine, l'isopropylamine, la diisopropylamine, la n-butylamine, la di-n-butylamine, la tri-n-butylamine, la di-isobutylamine, le 2-aminobutane, la 2-éthylhexylamine, la di-2-éthylhexylamine, la cyclohexylamine, la dicyclohexylamine, la diméthylaminopropylamine, la triéthylènediamine, le 1,8-diazabicyclo[5.4.0]undec-7-ène (DBU) et le 1,5-diazabicyclo[4.3.0]non-5-ène (DBN), particulièrement préférablement constitué par l'hydroxyde de sodium, l'hydroxyde de potassium, l'hydroxyde de lithium, l'hydroxyde de magnésium, l'éthanolate de tétra-(n-butyl) ammonium, le méthanolate de tétra-(n-butyl)ammonium et l'isopropylate de tétra-(n-butyl) ammonium.

14. Acide de Bronsted (C) n-protoné selon l'une quelconque des revendications 10 à 13, l'au moins un acide de Bronsted (E'H) étant choisi dans le groupe constitué par les acides sulfoniques fluorés aliphatiques, les acides sulfoniques fluorés aromatiques, l'acide trifluorométhanesulfonique, l'acide perchlorique, l'acide chlorhydrique, l'acide bromhydrique, l'acide iodhydrique, l'acide fluorosulfurique, le bis(trifluorométhane)sulfonimide, l'acide hexafluoroantimonique, le pentacyanocyclopentadiène, l'acide picrique, l'acide sulfurique, l'acide nitrique, l'acide trifluoroacétique, l'acide méthanesulfonique, l'acide para-toluènesulfonique, les acides sulfoniques aromatiques et les acides sulfoniques aliphatiques, préférablement constitué par l'acide trifluorométhanesulfonique, l'acide perchlorique, l'acide chlorhydrique, l'acide bromhydrique, l'acide iodhydrique, l'acide fluorosulfurique, le bis(trifluorométhane)sulfonimide, l'acide hexafluoroantimonique, le pentacyanocyclopentadiène, l'acide picrique, l'acide sulfurique, l'acide nitrique, l'acide trifluoroacétique, l'acide méthanesulfonique, l'acide para-toluènesulfonique, l'acide méthanesulfonique et l'acide para-toluènesulfonique, particulièrement préférablement constitué par l'acide trifluorométhanesulfonique, l'acide perchlorique, l'acide chlorhydrique, l'acide bromhydrique, l'acide iodhydrique, le bis(trifluorométhane)sulfonimide, le pentacyanocyclopentadiène, l'acide sulfurique, l'acide nitrique et l'acide trifluoroacétique.

15. Acide sulfonique selon l'une quelconque des revendications 10 à 14, le degré de protolyse D étant de 0,8 à 1,8, préférablement de 1,1 à 1,7.
